(19) Europäisches Patentamt
European Patent Office
Office européen des brevets

(11) EP 3 865 488 A1

(12) EUROPEAN PATENT APPLICATION
published in accordance with Art. 153(4) EPC

(43) Date of publication:
**18.08.2021 Bulletin 2021/33**

(21) Application number: **19897614.4**

(22) Date of filing: **11.12.2019**

(51) Int Cl.:
***C07D 498/04*** *(2006.01)* ***A61P 35/00*** *(2006.01)*
***A61K 31/519*** *(2006.01)*

(86) International application number:
**PCT/CN2019/124575**

(87) International publication number:
**WO 2020/125513 (25.06.2020 Gazette 2020/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **19.12.2018 CN 201811558752**

(71) Applicant: **Keythera (Suzhou) Pharmaceuticals Co. Ltd.**
**Suzhou, Jiangsu 215000 (CN)**

(72) Inventors:
- **DENG, Yongqi**
**Hong Kong (CN)**
- **SUN, Jian**
**Hong Kong (CN)**

(74) Representative: **Danner, Stefan**
**Danner BioPharm IP**
**Ludwigstraße 8**
**80539 München (DE)**

# (54) MACROCYCLIC COMPOUND AS CDK INHIBITOR, PREPARATION METHOD THEREFOR, AND USE THEREOF IN MEDICINE

(57) The present invention relates to a macrocyclic compound as a CDK inhibitor, a preparation method therefor and the use thereof in medicine. Specifically, the present invention relates to a novel macrocyclic compound represented by a general formula (I), a preparation method therefor, a pharmaceutical composition containing the compound, the use thereof as a therapeutic agent, particularly as a CDK inhibitor, and the use thereof in treating cancers, inflammation, viral infections, cardiac hypertrophy or HIV, wherein each substituent of the general formula (I) is the same as that defined in the description.

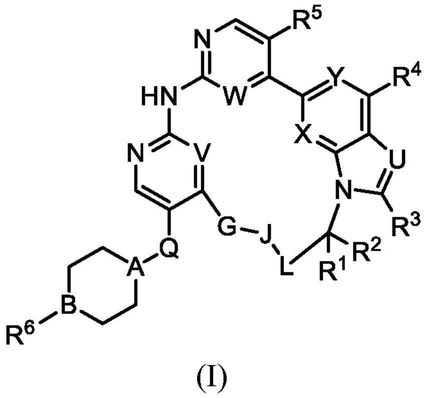

(I)

EP 3 865 488 A1

## Description

## FIELD OF THE INVENTION

The present invention belongs to the field of medicine, and relates to a novel macrocyclic compound of formula (I), a preparation method therefor, a pharmaceutical composition thereof, and an application thereof as a therapeutical agent, particularly CDK inhibitors for the treatment of hyperproliferative diseases.

## BACKGROUND OF THE INVENTION

In recent years, tumors have surpassed cardiovascular diseases and become the world's number one cause of death. The research of anti-tumor drugs thus has important academic and practical significance. Tumor cells are known to be over active and show continuous cell proliferation. , therefore, blocking cell cycle can effectively inhibit tumor growth. Cyclin dependent kinase (CDK) belongs to the serine/threonine protein kinase family, and is a key kinase involved in cell cycle regulation. There are currently 20 different CDKs reported. According to the differences in function, CDK can be divided into two main groups. One group is involved in the cell cycle regulation, mainly including CDK1, CDK2, CDK4, CDK6 etc. The other group is involved in the transcription regulation, mainly including CDK7, CDK8, CDK9, CDK10, CDK11 etc. In tumor cells, overexpression or overactivation of cyclin, decrease of CDK inhibitory mechanisms, and continuous activation of upstream division signals can all cause changes in CDK activity. Disregulation of CDK activity can directly or indirectly cause uncontrolled cell proliferation, genome instability (increased DNA mutations, chromosome deletion, etc.) and chromosomal instability (changes in the number of chromosomes), which is involved in the development of tumor. Since CDK activity is required for cell division, and CDK activity is often enhanced in tumor cells, CDKs have been considered as good targets for tumor and for other hyper-proliferative diseases for a while.

LY2835219 (also known as Bemaciclib or Abemaciclib), developed by Eli Lilly, is a drug for the treatment of breast cancer by inhibiting cyclin dependent kinases 4 and 6 (CDK4/6). Its structure is

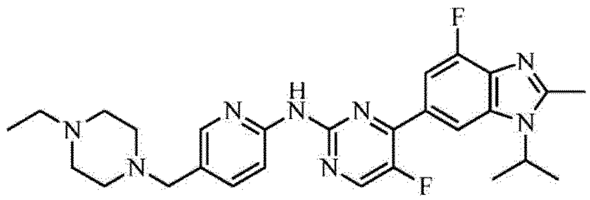

Phase I clinical trial data show that this drug alone has a good early efficacy on patients with metastatic breast cancer, especially on patients with hormone receptor-positive breast cancer, and the clinical benefit rate can reach 61%. This means that the patient's disease control duration exceeds 24 weeks, or the tumor size decreases by more than 30%. At present, the US FDA has approved Eli Lilly's Abemaciclib (VERZENIO) in combination with non-steroidal aromatase inhibitor (NSAI) letrozole or anastrozole as a first-line treatment of postmenopausal patients with hormone receptor (HR)-positive, human epidermal growth factor receptor 2 (HER2)-negative, advanced or metastatic breast cancer. Clinical research on non-small cell lung cancer has also begun.

PD0332991 (also known as Ibrance or Palbociclib) is another CDK4/6 inhibitor developed by Pfizer. Its structure is

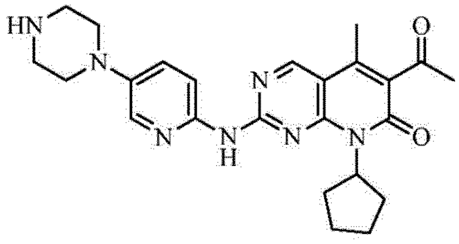

On February 3, 2015, the FDA approved PD0332991 in combination with letrozole for treating postmenopausal women with hormone receptor (HR)-positive, human epidermal growth factor receptor 2 (HER2)-negative, metastatic breast cancer through accelerated procedure.

CDK inhibitors can also be used to treat cardiovascular disorders such as restenosis and atherosclerosis, and other vascular disorders that are due to aberrant cell proliferation. Vascular smooth muscle proliferation and intimal

hyperplasia following balloon angioplasty are inhibited by over-expression of the cyclin dependent kinase inhibitor protein. Moreover, the purine CDK2 inhibitor CVT-313 (Ki = 95 nM) resulted in greater than 80% inhibition of neointima formation in rats.

CDK inhibitors can be used to treat diseases caused by a variety of infectious substances, including fungi, protozoan parasites such as Plasmodium falciparum, and DNA and RNA viruses.

Inhibition of CDK9 was recently linked to prevention of HIV replication and the discovery of new CDK biology thus continues to open up new therapeutic indications for CDK inhibitors.

CDKs are important in neutrophil-mediated inflammation, and CDK inhibitors promote the resolution of inflammation in animal models. Therefore, CDK inhibitors including CDK9 inhibitors can be used as anti-inflammatory agents.

Selective CDK inhibitors can be used to ameliorate the effects of various autoimmune disorders. The chronic inflammatory disease rheumatoid arthritis is characterized by synovial tissue proliferation; inhibition of synovial tissue proliferation should minimize inflammation and prevent joint destruction. In the rat arthritis model, joint swelling was substantially inhibited by treatment with adenovirus expressing a CDK inhibitor protein p16. CDK inhibitors are effective against other disorders of cell proliferation including psoriasis (characterized by keratinocyte hyperproliferation), glomerulonephritis, chronic inflammation and lupus.

The existing patent applications for CDK inhibitors include, for example, WO2015101293A1, WO2016015605A1, WO2016194831A1, WO2008079933A2 etc. It is still necessary to develop low-toxic and highly effective CDK inhibitors due to the huge market demand.

Macrocycles have been recognized as an important structural category in drug discovery. When having the same number of heavy atoms, a macrocyclic compound has less rotatable bonds than its acyclic counterparts, and this is a beneficial feature for oral bioavailability [Mallinson, J.; Collins, I. Macrocycles in new drug discovery. Future Med. Chem. 2012, 4, 1409-1438]. As a result, a macrocyclic compound has more conformational restriction than its acyclic counterparts, and this may bring higher target binding selectivity and improved oral bioavailability.

The development of natural macrocyclic compounds has produced several anti-tumor drugs, which have been approved for clinical use or have reached late-stage clinical development, such as a mTOR inhibitor Torisel® (temsirolimus) [Kwitkowski VE, Prowell TM, Ibrahim A et al. FDA approval summary: temsirolimus as treatment for advanced renal cell carcinoma. Oncologist 15(4), 428-435 (2010)], tubulin stabilizer Ixempra® (Ixabepilone) and Hsp90 inhibitor 17-allylamino-geldanamycin [Mcdonald E, Workman P, Jones K. Inhibitors of the HSP90 molecular chaperone: attacking the master regulator in cancer. Curr. Top. Med. Chem. 6(11), 1091-1107 (2006)]. Several synthetic macrocyclic compounds have entered clinical development stage, such as dual JAK2/FLT3 inhibitor pacritinib, which is currently undergoing phase II clinical trial [Hart S, Goh KC, Novotny-Diermayr V et al. SB 1518, a novel macrocyclic pyrimidinebased JAK2 inhibitor for the treatment of myeloid and lymphoid malignancies. Leukemia 25(11), 1751-1759 (2011)], a CDK2/JAK2/FLT3 inhibitor SB1317 which is currently undergoing phase II clinical trial [William AD, Lee ACH, Goh KC et al. Discovery of kinase spectrum selective macrocycle (16E)-14-methyl-20oxa5,7,14,26-tetraazatetracyclo[19.3.1.1(2,6).1(8,12)]heptacosa1(25 ),2(26),3,5,8(27),9,11,16,21,23-decaene (SB1317/TG02), a potent inhibitor of cyclin dependent kinases (CDKs), janus kinase 2 (JAK2), and Fms-like tyrosine kinase-3 (FLT3) for the treatment of cancer. J. Med. Chem. 55(1), 169-196 (2012)], and Cilengitide (a synthetic cyclic peptide for treating glioblastoma, which is currently undergoing phase III clinical trial) [Stupp R, Van Den Bent MJ, Erridge SC et al. Cilengitide in newly diagnosed glioblastoma with MGMT promoter methylation: Protocol of a multicenter, randomized, open-label, controlled phase III trial (CENTRIC). J. Clin. Oncol. 28(15s), TPS152 (2010)]. Pan-CDK inhibitor compound M is another example of a synthetic macrocyclic compound proposed as a developing candidate [Hirai H, Takahashi-Suziki I, Shimomura T et al. Potent anti-tumor activity of a macrocycle-quinoxalinone class pan-Cdk inhibitor in vitro and in vivo. Invest. New Drugs 29(4), 534-543 (2011)].

## SUMMARY OF THE INVENTION

The object of the present invention is to provide a compound with excellent CDK inhibitory activity. In order to achieve this object, the inventor has repeatedly conducted serious studies, and obtained a novel macrocyclic compound with excellent CDK inhibitory activity through an intramolecular cyclization reaction, thereby completing the present invention.

The present invention more particularly relates to a following compound of formula (I):

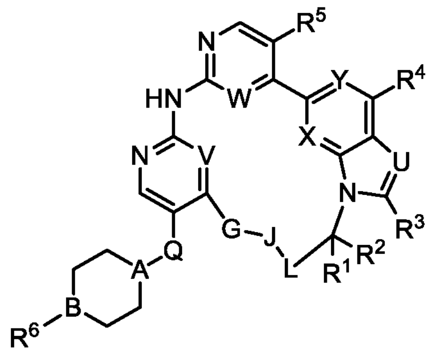

or a tautomer, mesomer, racemate, enantiomer, diastereomer thereof, or mixture thereof, or a pharmaceutically acceptable salt thereof, wherein:

A and B are each independently selected from the group consisting of CH and N atom;
U, V, W, X and Y are each independently selected from the group consisting of CH and N atom;
Q is selected from the group consisting of a bond, $C_{1-4}$ alkylene and -C(=O)-, wherein the $C_{1-4}$ alkylene is optionally substituted by one or more substituents selected from the group consisting of $C_{1-4}$ alkyl, halo$C_{1-4}$ alkyl, $C_{1-4}$ alkoxy, halo$C_{1-4}$ alkoxy, halogen, amino, nitro, hydroxy and cyano;
G is selected from the group consisting of a bond, -O-, -N($R^7$)-, -C(=O)-, $C_{1-4}$ alkylene, $C_{2-4}$ alkenyl, -S-, -SO-, -$SO_2$- and 3 to 6 membered heterocyclyl, wherein the $C_{1-4}$ alkylene, $C_{2-4}$ alkenyl and 3 to 6 membered heterocyclyl are each independently optionally substituted by one or more substituents selected from the group consisting of $C_{1-4}$ alkyl, halo$C_{1-4}$ alkyl, $C_{1-4}$ alkoxy, halo$C_{1-4}$ alkoxy, halogen, amino, nitro, hydroxy and cyano;
J is selected from the group consisting of a bond, -O-, -N($R^7$)-, -C(=O)-, $C_{1-4}$ alkylene, $C_{2-4}$ alkenyl, -S-, -SO-, -$SO_2$- and 3 to 6 membered heterocyclyl, wherein the $C_{1-4}$ alkylene, $C_{2-4}$ alkenyl and 3 to 6 membered heterocyclyl are each independently optionally substituted by one or more substituents selected from the group consisting of $C_{1-4}$ alkyl, halo$C_{1-4}$ alkyl, $C_{1-4}$ alkoxy, halo$C_{1-4}$ alkoxy, halogen, amino, nitro, hydroxy and cyano;
L is selected from the group consisting of a bond, -C(=O)-, $C_{1-4}$ alkylene, $C_{2-4}$ alkenyl, -S-, -SO-, -$SO_2$- and 3 to 6 membered heterocyclyl, wherein the $C_{1-4}$ alkylene, $C_{2-4}$ alkenyl and 3 to 6 membered heterocyclyl are each independently optionally substituted by one or more substituents selected from the group consisting of $C_{1-4}$ alkyl, halo$C_{1-4}$ alkyl, $C_{1-4}$ alkoxy, halo$C_{1-4}$ alkoxy, halogen, amino, nitro, hydroxy and cyano;
$R^1$ is selected from the group consisting of H atom, $C_{1-4}$ alkyl, cyano, $C_{2-4}$ alkenyl and $C_{2-4}$ alkynyl, wherein the $C_{1-4}$ alkyl, $C_{2-4}$ alkenyl and $C_{2-4}$ alkynyl are each independently optionally substituted by one or more substituents selected from the group consisting of halogen, amino, nitro, hydroxy and cyano;
$R^2$ is selected from the group consisting of H atom and $C_{1-4}$ alkyl, wherein the $C_{1-4}$ alkyl is optionally substituted by one or more substituents selected from the group consisting of halogen, amino, nitro, hydroxy and cyano;
or, $R^1$ and $R^2$ together with the atom to which they are attached form a $C_{3-6}$ cycloalkyl or 3 to 6 membered heterocyclyl, wherein the $C_{3-6}$ cycloalkyl or 3 to 6 membered heterocyclyl are each independently optionally substituted by one or more substituents selected from the group consisting of $C_{1-4}$ alkyl, halo$C_{1-4}$ alkyl, $C_{1-4}$ alkoxy, halo$C_{1-4}$ alkoxy, halogen, amino, nitro, hydroxy and cyano;
$R^3$ and $R^6$ are each independently selected from the group consisting of H atom and $C_{1-4}$ alkyl, wherein the alkyl is optionally substituted by one or more substituents selected from the group consisting of $C_{1-4}$ alkoxy, halo$C_{1-4}$ alkoxy, halogen, amino, nitro, hydroxy and cyano;
$R^4$ and $R^5$ are each independently selected from the group consisting of H atom, $C_{1-4}$ alkyl, halogen, hydroxy and $C_{1-4}$ alkoxy, wherein the $C_{1-4}$ alkyl and $C_{1-4}$ alkoxy are each independently optionally substituted by one or more substituents selected from the group consisting of halogen, amino, nitro, hydroxy and cyano; and
$R^7$ is selected from the group consisting of H atom and $C_{1-4}$ alkyl, wherein the alkyl is optionally substituted by one or more substituents selected from the group consisting of $C_{1-4}$ alkoxy, halo$C_{1-4}$ alkoxy, halogen, amino, nitro, hydroxy and cyano.
In a preferred embodiment, in the compound of formula (I), -G-J-L- is selected from the group consisting of -NH-$CH_2$-$CH_2$-, -O-$CH_2$-$CH_2$-, -NH-C(=O)-$CH_2$- and -C(=O)-NH-$CH_2$-.

In another preferred embodiment, in the compound of formula (I), A and B are each independently an N atom.
In another preferred embodiment, in the compound of formula (I), Q is a bond or methylene.
In another preferred embodiment, in the compound of formula (I), U is selected from the group consisting of CH and N atom.

In another preferred embodiment, in the compound of formula (I), V, X and Y are each independently CH.

In another preferred embodiment, in the compound of formula (I), W is an N atom.

In another preferred embodiment, in the compound of formula (I), $R^1$ and $R^2$ are each independently selected from the group consisting of H atom and $C_{1-4}$ alkyl, or $R^1$ and $R^2$ together with the atom to which they are attached form a $C_{3-6}$ cycloalkyl.

In another preferred embodiment, in the compound of formula (I), $R^3$ and $R^6$ are each independently a $C_{1-4}$ alkyl, and preferably $R^3$ is a methyl and $R^6$ is an ethyl.

In another preferred embodiment, in the compound of formula (I), $R^4$ and $R^5$ are each independently a halogen, and preferably an F atom.

In another preferred embodiment, in the compound of formula (I), $R^7$ is a H atom.

Typical compounds of the present invention include, but are not limited to:

| Example No. | Structure | Chemical name |
|---|---|---|
| 1 | 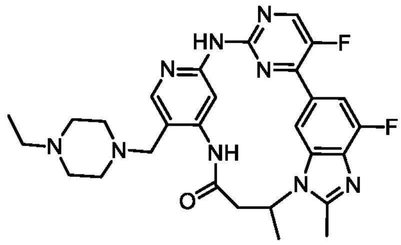 | $4^5$-((4-Ethylpiperazin-1-yl)methyl)-$1^4$,$2^5$-difluoro-$1^2$,8-dimethyl-$1^1$H-3,5-diaza-1(6 ,1)-benzo[*d*]imidazola-2 (4,2)-pyrimidina-4(2,4)-pyridinacyclooctaphan-6-one |
| 2 | 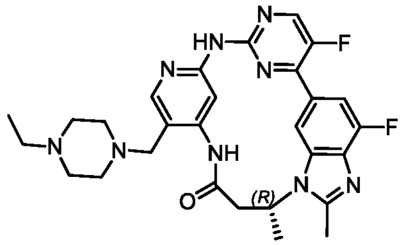 | (R)-$4^5$-((4-Ethylpiperazin-1-yl)methyl)-1 $^4$,$2^5$-difluoro-$1^2$,8-dimethyl-$1^1$H-3,5-diaza -1(6,1)-benzo[*d*]imidazola-2 (4,2)-pyrimi dina-4(2,4)-pyridinacyclooctaphan-6-one |
| 3 | 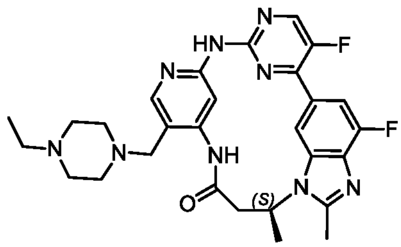 | (S)-$4^5$-((4-Ethylpiperazin-1-yl)methyl)-$1^4$ ,$2^5$-difluoro-$1^2$,8-dimethyl-$1^1$H-3,5-diaza-1(6,1)-benzo[*d*]imidazola-2 (4,2)-pyrimid ina-4(2,4)-pyridinacyclooctaphan-6-one |
| 4 | 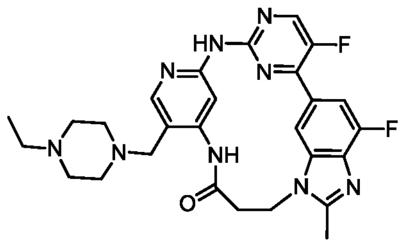 | $4^5$-((4-Ethylpiperazin-1-yl)methyl)-$1^4$,$2^5$-difluoro-$1^2$-methyl-$1^1$H-3,5-diaza-1(6,1)-benzo[d]imidazola-2(4,2)-pyrimidina-4(2 ,4)- pyri dinacycl ooctaphan-6-one |
| 5 | 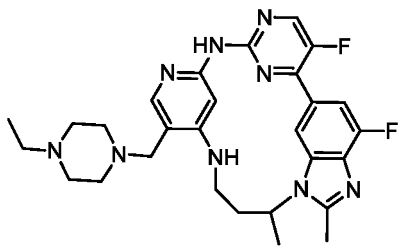 | $4^5$-((4-Ethylpiperazin-1-yl)methyl)-$1^4$,$2^5$-difluoro-$1^2$,8-dimethyl-$1^1$H-3,5-diaza-1(6 ,1)-benzo[d]imidazola-2 (4,2)-pyrimidina-4(2,4)-pyridinacyclooctaphane |

(continued)

| Example No. | Structure | Chemical name |
|---|---|---|
| 6 | 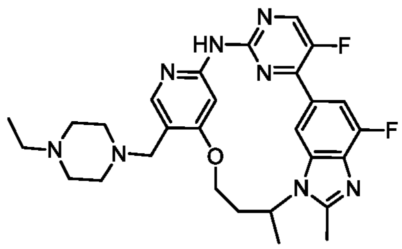 | $4^5$-((4-Ethylpiperazin-1-yl)methyl)-$1^4$,$2^5$-difluoro-$1^2$,8-dimethyl-$1^1$H-5-oxa-3-aza-1(6,1)-benzo[d]imidazola-2 (4,2)-pyrimid ina-4(2,4)-pyridinacyclooctaphane |
| 7 | 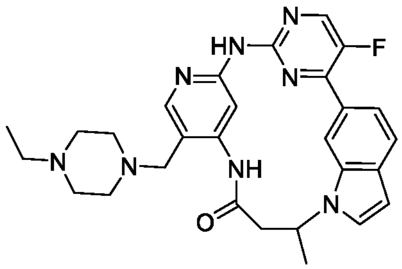 | $4^5$-((4-Ethylpiperazin-1-yl)methyl)-$2^5$-flu oro-8-methyl-$1^1$H-3,5-diaza-1(6,1)-indol a-2(4,2)-pyrimidina-4 (2,4)-pyridinacyclo octaphan-6-one |
| 8 | 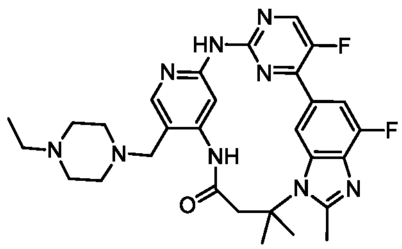 | $4^5$-((4-Ethylpiperazin-1-yl)methyl)-$1^4$,$2^5$-difluoro-$1^2$,8,8-trimethyl-$1^1$H-3,5-diaza-1 (6,1)-benzo[d]imidazola-2 (4,2)-pyrimidi na-4(2,4)-pyridinacyclooctaphan-6-one |
| 9 | 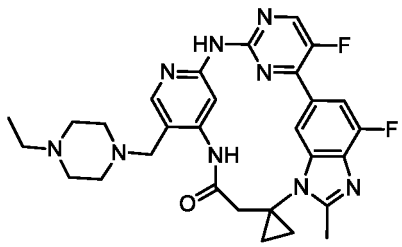 | 5'-((4-Ethylpiperazin-1-yl)methyl)-4',5'-d ifluoro-2'-methylspiro[cyclopropane-1,8'-3,5-diaza-1(6,1)-benzo[d] imidazola-2(4,2 )-pyrimidina-4(2,4)-pyridinacyclooctaph an]-6'-one |
| 10 | 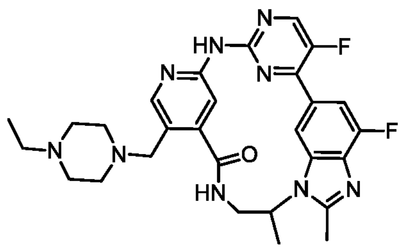 | $4^5$-((4-Ethylpiperazin-1-yl)methyl)-$1^4$,$2^5$-difluoro-$1^2$, 8-dimethyl-$1^1$H-3,6-diaza-1(6 ,1)-benzo[d]imidazola-2 (4,2)-pyrimidina-4(2,4)-pyridinacyclooctaphan-5-one |
| 11 | 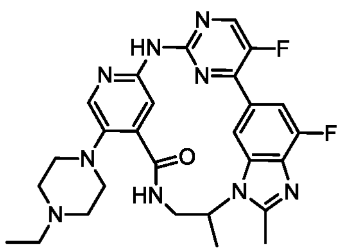 | $4^5$-(4-Ethylpiperazin-1-yl)-$1^4$,$2^5$-difluoro-$1^2$,8-dimethyl-$1^1$H-3,6-diaza-l(6,1)-benz o[d]imidazola-2(4,2)-pyrimidina-4 (2,4)-p yridinacyclooctaphan-5-one |

or a tautomer, mesomer, racemate, enantiomer, diastereomer thereof, or mixture thereof, or a pharmaceutically acceptable salt thereof.

The present invention also relates to a method for preparing the compound of formula (I), comprising a step of:

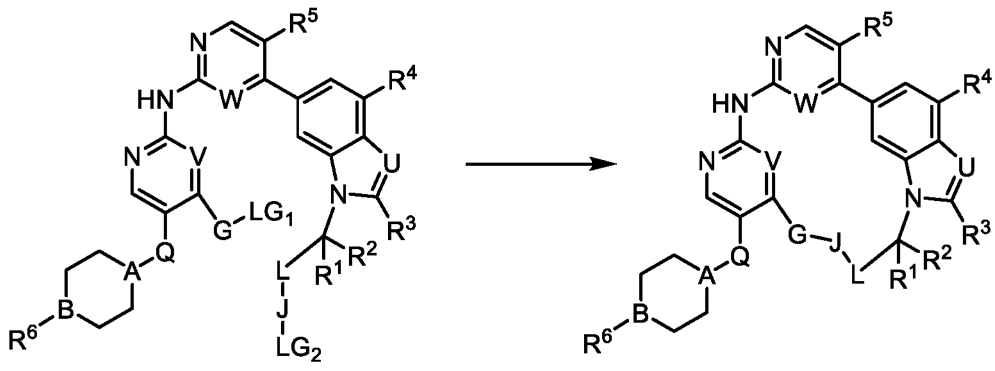

(IA) (I)

subjecting a compound of formula (IA) to an intramolecular cyclization reaction at the presence of a condensing agent to obtain the compound of formula (I), wherein:

$LG_1$ and $LG_2$ are each independently a leaving group;
A, B, U, V, W, G, J, L, and $R^1$ to $R^6$ are as defined in formula (I).

In a preferred embodiment, the condensing agent is selected from the group consisting of 2-(7-azabenzotriazol-1-yl)-*N*,*N*,*N'*,*N'*-tetramethyluronium hexafluorophosphate (HATU), 1-(3-dimethylaminopropyl)-3-ethylcarbodiimide hydrochloride, *N*,*N'*-dicyclohexylcarbodiimide, *N*,*N'*-diisopropylcarbodiimide, *O*-(benzotriazol-1-yl)-*N*,*N*,*N'*,*N'*-tetramethyluronium tetrafluoroborate, 1-hydroxybenzotriazole, 1-hydroxy-7-azobenzotriazole, *O*-(benzotriazol-1-yl)-*N*,*N*,*N'*,*N'*-tetramethyluronium hexafluorophosphate, 2-(7-azobenzotriazole)-*N*,*N*,*N'*,*N'*-tetramethyluronium hexafluorophosphate, benzotriazol-1-yloxytris(dimethylamino)phosphonium hexafluorophosphate and benzotriazol-1-yl-oxytripyrrolidinophosphonium hexafluorophosphate, and preferably 2-(7-azabenzotriazol-1-yl)-*N*,*N*,*N'*,*N'*-tetramethyluronium hexafluorophosphate (HATU).

In another preferred embodiment, $LG_1$ and $LG_2$ are each independently selected from the group consisting of H atom, OH, halogen, mesylate, triflate and tosylate; preferably, $LG_1$ and $LG_2$ are each independently selected from the group consisting of H atom, OH, Cl atom and Br atom; and more preferably, $LG_1$ is a H atom and $LG_2$ is OH. In another preferred embodiment, G is -NH-, $LG_1$ is a H atom, J is -C(=O)-, and -$LG_2$ is OH.

The present invention also relates to a pharmaceutical composition comprising a therapeutically effective amount of the compound of formula (I), or a tautomer, mesomer, racemate, enantiomer, diastereomer thereof, or mixture thereof, or a pharmaceutically acceptable salt thereof, and pharmaceutically acceptable carrier(s), diluent(s) or excipient(s).

In a preferred embodiment, the pharmaceutical composition also comprises one or more additional anti-tumor agents, anti-inflammatory agents, immunosuppressants and/or immune checkpoint inhibitors.

In another preferred embodiment, the pharmaceutical composition also comprises one or more of: PTK inhibitors, cyclosporin A, CTLA4-Ig, antibodies selected from the group consisting of anti-ICAM-3, anti-IL-2 receptor, anti-CD45RB, anti-CD2, anti-CD3, anti-CD4, anti-CD80, anti-CD86 and monoclonal antibody OKT3, CVT-313, agents blocking the interaction between CD40 and gp39, fusion proteins constructed from CD40 and gp39, inhibitors of NF-κB function, non-steroidal anti-inflammatory agents, steroids, gold compounds, FK506, mycophenolate mofetil, cytotoxic drugs, TNF-α inhibitors, anti-TNF antibodies or soluble TNF receptors, TNFα, TRAIL, HDAC inhibitors, Gleevec and other inhibitors of signal transduction pathways involved in cell proliferation, inhibitors of cellular responses to hypoxia, rapamycin, leflunomide, cyclooxygenase-2 inhibitors, paclitaxel, cisplatin, carboplatin, doxorubicin, carminomycin, daunorubicin, aminopterin, methotrexate, methopterin, mitomycin C, ascidianin 743, porfiromycin, 5-fluorouracil, 6-mercaptopurine, gemcitabine, cytosine arabinoside, podophyllotoxin, etoposide, etoposide phosphate, teniposide, melphalan, vinblastine, vincristine, leurosidine, epothilone, vindesine, vinleurosine, PD-1, PDL-1 or derivatives thereof.

The present invention also relates to a use of the compound of formula (I), or a tautomer, mesomer, racemate, enantiomer, diastereomer thereof, or mixture thereof, or a pharmaceutically acceptable salt thereof, or the pharmaceutical composition comprising the same in the preparation of a medicament for treating a CDK-related disease.

The present invention also relates to the compound of formula (I), or a tautomer, mesomer, racemate, enantiomer, diastereomer thereof, or mixture thereof, or a pharmaceutically acceptable salt thereof,

omer, diastereomer thereof, or mixture thereof, or a pharmaceutically acceptable salt thereof, or the pharmaceutical composition comprising the same, for use as a medicament.

The present invention also relates to the compound of formula (I), or a tautomer, mesomer, racemate, enantiomer, diastereomer thereof, or mixture thereof, or a pharmaceutically acceptable salt thereof, or the pharmaceutical composition comprising the same, for use in treating a CDK-related disease.

The present invention also relates to a method for treating a CDK-related disease, comprising a step of administrating to a patient in need thereof a therapeutically effective dose of the compound of formula (I), or a tautomer, mesomer, racemate, enantiomer, diastereomer thereof, or mixture thereof, or a pharmaceutically acceptable salt thereof, or the pharmaceutical composition comprising the same.

In a preferred embodiment, the CDK-related disease is selected from the group consisting of cancer, inflammation, viral infection, cardiac hypertrophy and HIV

In a more preferred embodiment, the cancer is selected from the group consisting of bladder cancer, head and neck cancer, breast cancer, stomach cancer, ovarian cancer, colon cancer, lung cancer, brain cancer, laryngeal cancer, lymphatic system cancer, hematopoietic system cancer, urogenital cancer, genitourinary tract cancer, ovarian cancer, prostate cancer, gastric cancer, bone cancer, small cell lung cancer, glioma, colorectal cancer and pancreatic cancer;
the inflammation is related to rheumatoid arthritis, lupus, type 1 diabetes, diabetic nephropathy, multiple sclerosis, glomerulonephritis, chronic inflammation and organ transplant rejection;
the viral infection is related to HIV virus, human papilloma virus, herpes virus, pox virus, Epstein-Barr virus, Sindbis virus or adenovirus.

The compound according to the present invention can be administered orally, sublingually, parenterally, subcutaneously, intramuscularly, intravenously, transdermally, locally or rectally.

In the pharmaceutical compound of the present invention, for oral, sublingual, parenteral, subcutaneous, intramuscular, intravenous, transdermal, local or rectal administration, the active ingredient can be mixed with a conventional pharmaceutical carrier, and administered to animal or human in unit forms of administration. Suitable unit forms of administration include oral forms such as tablets, gel capsules, powders, granules and oral solutions or suspensions, sublingual or buccal forms for administration, parenteral, subcutaneous, intramuscular, intravenous, intranasal or intraocular forms of administration and rectal forms of administration.

When a solid composition is prepared into the form of tablets, the principal active ingredient is mixed with a pharmaceutical carrier such as gelatin, starch, lactose, magnesium stearate, talc, gum arabic and the like. The tablets can be coated with sucrose or other suitable materials, or else treated in such a way that they have an extended or delayed activity and continuously releases a predetermined amount of active ingredient.

A gel capsule preparation can be obtained by mixing the active ingredient with a diluent followed by pouring the resulting mixture into soft or hard capsules.

A preparation in the form of a syrup or elixir can comprise the active ingredient in combination with a sweetening agent, preservative, as well as a flavour-producing agent and appropriate colorant.

Powders or granules dispersible in water can comprise the active ingredient mixed together with dispersing agents, wetting agents, or suspending agents, as well as taste correctors or sweetening agents.

Suppository is used for rectal administration, which is prepared with binding agents melting at rectal temperature such as cocoa butter or polyethylene glycols.

Aqueous suspension, isotonic saline solution or sterile injectable solution (comprising pharmacologically compatible dispersing agents and/or wetting agents) is used for parenteral, intranasal or intraocular administration.

The active ingredient can also be formulated as microcapsules, possibly with one or more additive carriers.

The compound of the present invention can be used at a dose between 0.01 mg and 1000 mg per day, and adminstered in a single dose per day or in several doses throughout the day, for example, twice a day in equal doses. The daily dose administered is advantageously between 0.1 mg and 100 mg, and even more advantageously between 2.5 mg and 50 mg. It may be necessary to use doses exceeding these ranges, of which those skilled in the art will themselves be aware.

In a particular embodiment of the present invention, the pharmaceutical composition can also be formulated for topical administration. It can be introduced in forms commonly known for this type of administration (*i.e.*, especially lotion, foam, gel, dispersion, spray), and such forms comprise excipients that particularly enable penetration of the skin so as to improve the properties and accessibility of the active ingredient. Besides the composition according to the present invention, these compositions typically further comprise a physiologically acceptable medium, which generally contains water or a solvent such as an alcohol, ether or ethylene glycol. The composition can also comprise surfactants, preservatives, stabilizers, emulsifiers, thickeners, other active ingredients producing a complementary effect or a possibly synergistic effect, trace elements, essential oils, perfumes, colorants, collagen, chemical or mineral filters.

Definitions

Unless otherwise stated, the terms used in the specification and claims have the meanings described below.

Within the meaning of the present invention, the term "stereoisomer" refers to a geometric isomer (or configuration isomer) or optical isomer.

Geometric isomer results from different position of the substituents on a double bond which can then have a Z or E configuration, also called cis or trans configuration.

Optical isomers result in particular from the different spatial position of the substituents on a carbon atom comprising four different substituents. This carbon atom then constitutes a chiral center or asymmetric center. Optical isomers include diastereomers and enantiomers. Optical isomers that are non-superimposable mirror images of each other are called "enantiomers". Optical isomers that are not superimposable mirror images of each other are called "diastereomers".

A mixture containing equal amounts of two individual enantiomeric forms of opposite chirality is called a "racemic mixture".

Within the meaning of the present invention, the term "tautomer" refers to a constitutional isomer of the compound obtained by prototropie (i.e. by migration of a hydrogen atom and change of location of a double bond). The different tautomers of a compound are generally interconvertible and present in equilibrium in solution, in proportions that can vary depending on the solvent used, the temperature or the pH.

In the present invention, "pharmaceutically acceptable" is understood to mean that which is useful in the preparation of a pharmaceutical composition which is generally safe, non-toxic, and neither biologically nor otherwise undesirable and which is acceptable for veterinary and human pharmaceutical use.

In the present invention, the "pharmaceutically acceptable salt" of a compound is understood to refer to salts, which are pharmaceutically acceptable, as defined herein, and which possess the desired pharmacological activity of the parent compound. Such salts include:

(1) acid addition salts formed with inorganic acids such as hydrochloric acid, hydrobromic acid, sulfuric acid, nitric acid, phosphoric acid and the like; or acid addition salts formed with organic acids such as acetic acid, benzenesulfonic acid, benzoic acid, camphorsulfonic acid, citric acid, ethylsulfonic acid, fumaric acid, glucoheptonic acid, gluconic acid, glutamic acid, glycolic acid, hydroxynaphtoic acid, 2-hydroxylethylsulfonic acid, lactic acid, maleic acid, malic acid, mandelic acid, methanesulfonic acid, muconic acid, 2-naphthalenesulfonic acid, propionic acid, salicylic acid, succinic acid, dibenzoyl-L-tartaric acid, tartaric acid, *p*-toluenesulfonic acid, trimethylacetic acid, trifluoroacetic acid and the like; and

(2) salts formed when an acidic proton present in parent compound is either replaced by a metal ion such as an alkali metal ion (e.g., $Na^+$, $K^+$ or $Li^+$), an alkaline earth metal ion (e.g. $Ca^{2+}$ or $Mg^{2+}$), or an aluminum ion; or coordinates with an organic base or inorganic base. Acceptable organic bases include diethanolamine, ethanolamine, N-methylglucamine, triethanolamine, tromethamine and the like. Acceptable inorganic bases include aluminum hydroxide, calcium hydroxide, potassium hydroxide, sodium carbonate and sodium hydroxide.

In the present invention, the term "halogen" refers to fluorine, bromine, chlorine or iodine atom.

The term "$C_{1-4}$ alkyl" refers to a saturated, linear or branched hydrocarbon chain comprising 1 to 4 carbon atoms. Representative examples include, but are not limited to methyl, ethyl, *n*-propyl, isopropyl, *n*-butyl, *iso*-butyl, *sec*-butyl, *tert-butyl* groups.

The term "$C_{1-4}$ alkylene" refers to a divalent hydrocarbon chain comprising 1 to 4 carbon atoms. Representative examples include, but are not limited to $-CH_2-$, $-CH_2-CH_2-$, $-CH_2-CH_2-CH_2-$, $-CH_2-CH_2-CH_2-CH_2-$ and the like.

The term "$C_{2-4}$ alkenyl" refers to a straight or branched hydrocarbon chain having at least one double bond and 2 to 4 carbon atoms. Representative examples include, but are not limited to ethenyl, propenyl, butenyl and the like.

The term "$C_{2-4}$ alkynyl" refers to a straight or branched hydrocarbon chain having at least one triple bond and 2 to 4 carbon atoms. Representative examples include, but are not limited to ethynyl, propynyl, butynyl and the like.

The term "$C_{1-4}$ alkoxy" refers to an -O-($C_{1-4}$ alkyl) group, wherein the $C_{1-4}$ alkyl is as defined above. Non-limiting examples include methoxy, ethoxy, propyloxy, butoxy and the like.

The term "halo$C_{1-4}$ alkyl" refers to a $C_{1-4}$ alkyl group substituted by one or more halogens, wherein the $C_{1-4}$ alkyl and halogen are as defined above.

The term "halo$C_{1-4}$ alkoxy" refers to a $C_{1-4}$ alkoxy group substituted by one or more halogens, wherein the $C_{1-4}$ alkoxy and halogen are as defined above.

The term "$C_{3-6}$ cycloalkyl" refers to a saturated or partially unsaturated monocyclic hydrocarbon system having 3 to 6 carbon atoms. Representative examples include, but are not limited to cyclohexyl, cyclopentyl, cyclobutyl, cyclopropyl, cyclohexenyl and the like.

The term "3 to 6 membered heterocyclyl" refers to a saturated or partially unsaturated monocyclic hydrocarbon

system having 3 to 6 ring atoms, wherein 1 to 3 ring atoms are heteroatoms selected from the group consisting of N, O and $S(O)_m$ (wherein m is 0, 1 or 2). Representative examples include, but are not limited to pyrrolidinyl, imidazolyl, tetrahydrofuranyl, tetrahydropyranyl, tetrahydrothienyl, dihydroimidazolyl, dihydrofuranyl, dihydropyrazolyl, dihydropyrrolyl, piperidinyl, piperazinyl, morpholinyl and the like.

The term "hydroxy" refers to an -OH group.

The term "nitro" refers to a $-NO_2$ group.

The term "amino" refers to a $-NH_2$ group.

The term "cyano" refers to a -CN group.

The term "a bond" refers to a covalent bond represented by "-".

The term "leaving group" refers to a chemical group that can be easily displaced by a nucleophile during nucleophilic substitution reaction, such as H atom, OH, halogen atom (such as chlorine or bromine atom) or sulfonate. The sulfonate can in particular be a mesylate ($-OS(O_2)-CH_3$), triflate ($-OS(O)_2-CF_3$) or tosylate ($-OS(O)_2-(p\text{-}Me\text{-}C_6H_4)$).

"Optional" or "optionally" means that the event or circumstance described subsequently can, but need not, occur, and such a description includes the situation in which the event or circumstance does or does not occur. For example, "the heterocyclyl optionally substituted by an alkyl" means that an alkyl group can be, but need not be, present, and such a description includes the situation of the heterocyclyl being substituted by an alkyl and the heterocyclyl being not substituted by an alkyl.

"Substituted" refers to one or more hydrogen atoms in a group, preferably up to 5, and more preferably 1 to 3 hydrogen atoms, independently substituted by a corresponding number of substituents. It goes without saying that the substituents only exist in their possible chemical position. The person skilled in the art is able to determine whether the substitution is possible or impossible by experiments or theory without excessive effort. For example, the combination of amino or hydroxy having free hydrogen and carbon atoms having unsaturated bonds (such as olefinic) may be unstable.

A "pharmaceutical composition" refers to a mixture of one or more of the compounds according to the present invention or physiologically/pharmaceutically acceptable salts or prodrugs thereof with other chemical components, and other components such as physiologically/pharmaceutically acceptable carriers and excipients. The purpose of the pharmaceutical composition is to facilitate administration of a compound to an organism, which is conducive to the absorption of the active ingredient so as to show biological activity.

## DETAILED DESCRIPTION OF THE INVENTION

By reading the following examples, the person skilled in the art will better understand the present invention. The following examples merely serve to illuminate the present invention.

In the examples of the present invention, the experiment methods that do not specify the specific conditions are generally conducted in accordance with conventional conditions, or in accordance with conditions recommended by the material or product manufacturers. The reagents without a specific source are commercially available conventional reagents.

The structures of the compounds were identified by nuclear magnetic resonance (NMR) and/or mass spectrometry (MS). NMR shifts ($\delta$) are given in $10^{-6}$ (ppm). The solvent for determination was deuterated-chloroform ($CDCl_3$), and the internal standard was tetramethylsilane (TMS). The following abbreviations are used: s for singlet, bs for broad singlet, d for doublet, t for triplet, qdt for quartet, m for multiplet or massive, dd for double of doublet, etc.

Liquid chromatograph-mass spectrometer: Agilent LCMS 1260/MSD6120, column: Agilent ZORBAX SB-C18, 2.1*50mm, 1.8 $\mu$m, mobile phase: A: $H_2O$ (0.1% FA), B: acetonitrile, gradient elution, 0.5 mL/min, 45.0 °C, ionization mode: API-ES, polarity: positive.

Nuclear magnetic resonance spectrometer: Bruker ARX-500 and Bruker ARX-400.

MTT detection instrument: Thermo Scientific Multiskan GO full-wavelength microplate reader.

Qingdao GF254 silica gel plate is used as the thin-layer silica gel chromatography (TLC) plate. The dimension of the silica gel plate used in TLC is 0.15 mm to 0.2 mm, and the dimension of the silica gel plate used in product purification is 0.4 mm to 0.5 mm.

Yantai Huanghai 200 to 300 mesh silica gel is generally used as a carrier for silica gel column chromatography.

Unless otherwise stated, the reactions were carried out under argon atmosphere or nitrogen atmosphere.

Unless otherwise stated, the solution refers to an aqueous solution.

Unless otherwise stated, the reaction temperature is room temperature.

The reaction process in the examples was monitored by thin layer chromatography (TLC).

Example 1

$4^5$-((4-Ethylpiperazin-1-yl)methyl)-$1^4$,$2^5$-difluoro-$1^2$,8-dimethyl-$1^1$H-3,5-diaza-1(6,1)-b enzo[d]imidazola-2(4,2)-pyrimidina-4(2,4)-pyridinacyclooctaphan-6-one 1

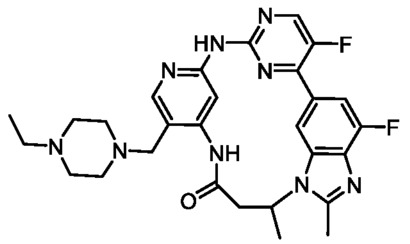

1

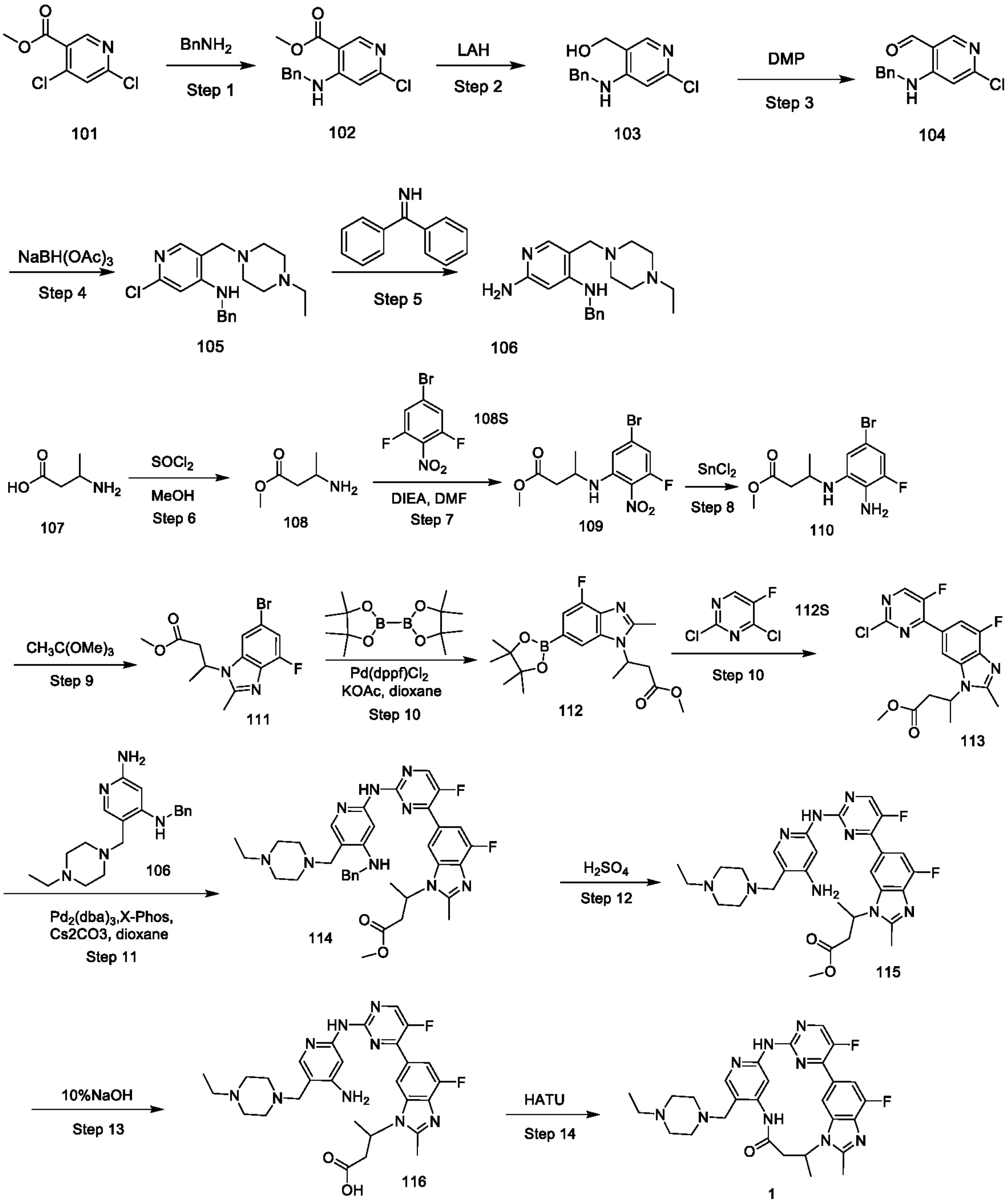


Step 1

Methyl 4-(benzylamino)-6-chloronicotinate **102**

Methyl 4,6-dichloronicotinate **101** (6 g, 29.1 mmol), benzylamine (3.4 g, 32 mmol) and triethylamine (9.5 g, 87.3 mmol) were dissolved in 30 mL of DMF and stirred at room temperature overnight. The reaction was monitored by TLC (petroleum ether: ethyl acetate = 5:1). After the reaction was completed, 200 mL of water was added. The reaction solution was stirred for 30 minutes and filtered, and the filter cake was washed with water and dried under reduced pressure. The title compound was obtained as a white solid (7.6 g, yield: 94.1%).

MS (ESI): 277.7 $[M+1]^+$.

Step 2

(4-(Benzylamino)-6-chloropyridin-3-yl)methanol **103**

Lithium aluminum hydride (2.1 g, 54.9 mmol) was suspended in 70 mL of tetrahydrofuran, and the solution was cooled to below -20°C, followed by the addition of methyl 4-(benzylamino)-6-chloronicotinate **102** (7.6 g, 27.4 mmol). The reaction solution was reacted at -20°C for 2 hours. The reaction was monitored by TLC (petroleum ether: ethyl acetate = 3:1). After the reaction was completed, 2.1 mL of water, 3.2 mL of 10% sodium hydroxide solution and 10.2 mL of water were added successively. The reaction solution was stirred for 30 minutes, dried and filtered, and the filtrate was concentrated to dryness by rotary evaporation. The title compound was obtained as a white solid (7.5 g, yield: 100%).
MS (ESI): 249.7 $[M+1]^+$.

Step 3

4-(Benzylamino)-6-chloronicotinaldehyde **104**

(4-(Benzylamino)-6-chloropyridin-3-yl)methanol **103** (7.5 g, 30.2 mmol) was dissolved in 100 mL of dichloromethane, and the solution was cooled to below 0°C, followed by the addition of DMP (Dess-Martin oxidant, 15.3 g, 36.3 mmol) in batches. The reaction solution was stirred at room temperature overnight. The reaction was monitored by TLC (ethyl acetate). After the reaction was completed, 100 mL of 10% NaOH solution was added. The reaction solution was stirred for 30 minutes and separated into two phases. The aqueous phase was extracted by dichloromethane. The organic phases were combined, dried and concentrated to dryness by rotary evaporation to obtain the crude product. The crude product was purified by column chromatography (PE:EA=3:1) to obtain the title compound as a yellow oil (6.5 g, yield: 87.1%).
MS (ESI):247.7 $[M+1]^+$.

Step 4

N-Benzyl-2-chloro-5-((4-ethylpiperazin-1-yl)methyl)pyridin-4-amine **105**

4-(Benzylamino)-6-chloronicotinaldehyde **104** (6.5 g, 26.3 mmol) and N-ethylpiperazine (3.6 g, 31.6 mmol) were dissolved in 50 mL of dichloromethane, and the solution was cooled to below 0°C, followed by the addition of sodium triacetoxyborohydride (8.3 g, 39.45 mmol) in batches. The reaction solution was stirred at room temperature overnight. The reaction was monitored by TLC (ethyl acetate). After the reaction was completed, 100 mL of saturated sodium bicarbonate solution was added. The reaction solution was stirred for 30 minutes and separated into two phases. The aqueous phase was extracted by dichloromethane (100 mL×3). The organic phases were combined, dried and concentrated to dryness by rotary evaporation to obtain the crude product. The crude product was purified by column chromatography (ethyl acetate: methanol = 10:1) to obtain the title compound as a colorless oil (7.8 g, yield: 85.9%).
MS (ESI):348.5 $[M+1]^+$.

Step 5

$N^4$-Benzyl-5-((4-ethylpiperazin-l-yl)methyl)pyridine-2,4-diamine **106**

The intermediate N-benzyl-2-chloro-5-((4-ethylpiperazin-1-yl)methyl)pylidin-4-amine **105** (6 g, 17.4 mmol), benzophenone imine (3.7 g, 20.8 mmol), sodium *tert*-butoxide (2.34 g, 24.36 mmol), $Pd_2(dba)_3$ (1.6 g, 1.74 mmol) and BINAP (3.24 g, 5.22 mmol) were dissolved in 100 mL of toluene under a nitrogen atmosphere. The reaction solution was reacted at 100°C for 14 hours. The reaction was monitored by TLC (DCM:MeOH = 10:1). After the reaction was completed, 100 mL of methyl *tert*-butyl ether was added. The reaction solution was stirred for 30 minutes, filtered, and washed, and the filtrate was concentrated to dryness by rotary evaporation to obtain the crude product. The crude product was dissolved in 50 mL of THF and 20 mL of methanol, followed by the addition of 10 mL of concentrated hydrochloric acid, and the reaction solution was stirred at room temperature for 2 hours. The reaction was monitored by TLC (dichloromethane: methanol = 10:1). After the reaction was completed, the reaction solution was concentrated to dryness by rotary evaporation. Saturated sodium bicarbonate solution was added, and the reaction solution was extracted by ethyl acetate. The aqueous phase was further extracted by dichloromethane. The organic phases were combined, dried and concentrated to dryness by rotary evaporation. The title compound was obtained as a white solid (3 g, yield: 52.9%).
MS (ESI):326.2 $[M+1]^+$.

Step 6

Methyl 3-aminobutanoate **108**

Thionyl chloride (8.4 mL, 116.4 mmol) was added dropwise to 40 mL of methanol and stirred for 1 hour at 0°C. 3-Aminobutyric acid **107** (4 g, 38.8 mmol) was added to the reaction system, which was then stirred at room temperature for 5 hours. The reaction was monitored by TLC (dichloromethane: methanol = 5:1). After the reaction was completed, the reaction solution was concentrated to dryness by rotary evaporation. Methanol was added, and then the solution was concentrated to dryness, and this process was repeated twice. The title compound was obtained as a colorless oil (5 g, yield: 100%).

Step 7

Methyl 3-((5-bromo-3-fluoro-2-nitrophenyl)amino)butanoate **109**

1,3-Difluoro-5-bromo-2-nitrobenzene (2 g, 8.4 mmol) and methyl 3-aminobutanoate **108** (0.98 g, 8.4mmol) were dissolved in 20 mL of DMF, followed by the addition of DIEA (3.25 g, 25.2 mmol). The reaction solution was stirred at room temperature overnight. The reaction was monitored by TLC (petroleum ether: ethyl acetate = 5:1). After the reaction was completed, 50 mL of water was added, and the reaction solution was extracted with ethyl acetate (3 × 25 mL). The organic phases were combined, washed with water, dried and concentrated to dryness by rotary evaporation to obtain the crude product. The crude product was purified by column chromatography (petroleum ether: ethyl acetate = 50:1) to obtain the title compound as a yellow solid (2.4 g, yield: 84.5%).

MS (ESI):336.1 $[M+1]^+$.

Step 8

Methyl 3-((2-amino-5-bromo-3-fluorophenyl)amino)butanoate **110**

Methyl 3-((5-bromo-3-fluoro-2-nitrophenyl)amino)butanoate **109** (2 g, 5.9 mmol) was dissolved in 15 mL of DMF, followed by the addition of stannous chloride (6.7 g, 29.8 mmol). The reaction solution was stirred at room temperature overnight. The reaction was monitored by TLC (petroleum ether: ethyl acetate = 5:1). After the reaction was completed, 50 mL of water was added, and the reaction solution was extracted with ethyl acetate (3 × 25 mL). The organic phases were combined, washed with water, dried and concentrated to dryness by rotary evaporation. The title compound was obtained as an orange oil (2.2 g, yield: 100%).

MS (ESI):306.2 $[M+1]^+$.

Step 9

Methyl 3-(6-bromo-4-fluoro-2-methyl-1H-benzo[d]imidazol-1-yl)butanoate **111**

Methyl 3-((2-amino-5-bromo-3-fluorophenyl)amino)butanoate **110** (2.2 g, 5.9 mmol) was dissolved in 9 mL of trimethyl orthoformate and 6 mL of acetic acid. The reaction solution was reacted at 90°C for 8 hours. The reaction was monitored by TLC (petroleum ether: ethyl acetate = 1:1). After the reaction was completed, the reaction solution was concentrated to dryness by rotary evaporation. Saturated sodium bicarbonate solution was added, and the reaction solution was extracted with ethyl acetate (3 × 15 mL). The organic phases were combined, washed with water, dried and concentrated to dryness by rotary evaporation to obtain the crude product. The crude product was dissolved in 10 mL of a solution of petroleum ether and ethyl acetate (1:10), stirred for one hour and filtered. The filter cake was washed with petroleum ether, and dried under reduced pressure to obtain the title compound as a white solid (1.2 g, yield: 61.2%).

MS (ESI):330.1 $[M+1]^+$.

Step 10

Methyl 3-(6-(2-chloro-5-fluoropyrimidin-4-yl)-4-fluoro-2-methyl-1H-benzo[d]imidazol-1-yl)bu tanoate **113**

Methyl 3-(6-bromo-4-fluoro-2-methyl-1H-benzo[d]imidazol-1-yl)butanoate **111** (2.5 g, 8 mmol), pinacol diborate (2.3 g, 9.1 mmol), potassium acetate (2.4 g, 24 mmol) and Pd(dppf)$Cl_2$ (326 mg, 0.4 mmol) were dissolved in 70 mL of dioxane under a nitrogen atmosphere. The reaction solution was reacted at 100°C for 2 hours. The reaction was monitored by HPLC. After the reaction was completed, the intermediate 2,4-dichloro-5-fluoropyrimidine (1.7 g, 10.4 mmol), cesium

carbonate (7.8 g, 24 mmol), Pd(dppf)$Cl_2$ (326 mg, 0.4 mmol) and 7 mL of water were added to the reaction solution. The reaction solution was reacted at 110°C for 14 hours. The reaction was monitored by LC-MS. When the raw material no longer decreased, the reaction was stopped. The reaction solution was concentrated to dryness by rotary evaporation. Water was added, and the solution was extracted with ethyl acetate (3 × 25 mL). The organic phases were combined, dried and concentrated to dryness by rotary evaporation to obtain the crude product. The crude product was purified by column chromatography (petroleum ether: ethyl acetate = 1:1) to obtain the title compound as a yellow solid (1 g, yield: 32.5%).

MS (ESI):381.1 $[M+1]^+$.

Step 11

Methyl 3-(6-(2-((4-(benzylamino)-5-((4-ethylpiperazin-1-yl)methyl)pyridin-2-yl)amino)-5-fluor opyrimidin-4-yl)-4-fluoro-2-methyl-1H-benzo[d]imidazol-1-yl)butanoate **114**

Methyl 3-(6-(2-chloro-5-fluoropyrimidin-4-yl)-4-fluoro-2-methyl-1H-benzo[d]imidazol-1-yl)bu tanoate **113** (0.7 g, 1.8 mmol), $N^4$-benzyl-5-((4-ethylpiperazin-1-yl)methyl)pyridine-2,4-diamine (0.72 g, 2.2 mmol), $Pd_2(dba)_3$ (82 mg, 0.09 mmol), 2-dicyclohexylphosphorus-2,4,6-triisopropylbiphenyl (X-phos) (128 mg, 0.27 mmol) and potassium carbonate (0.745 g, 5.4 mmol) were suspended in 70 mL of dioxane under a nitrogen atmosphere. The reaction solution was reacted at 100°C for 12 hours. The reaction was monitored by TLC (dichloromethane: methanol=5:1). After the reaction was completed, the reaction was stopped. The reaction solution was concentrated to dryness by rotary evaporation. Water was added, and the solution was extracted with dichloromethane (3 × 25 mL). The organic phases were combined, dried and concentrated to dryness by rotary evaporation to obtain the crude product. The crude product was purified by column chromatography (dichloromethane: methanol=5:1) to obtain the title compound as a brown solid (0.5 g, yield: 38.9%).

MS (ESI): 670.7 $[M+1]^+$.

Step 12

Methyl 3-(6-(2-((4-amino-5-((4-ethylpiperazin-1-yl)methyl)pyridin-2-yl)amino)-5-fluoropyrimi din-4-yl)-4-fluoro-2-methyl-1H-benzo[d]imidazol-1-yl)butanoate **115**

Methyl 3-(6-(2-((4-(benzylamino)-5-((4-ethylpiperazin-1-yl)methyl)pyridin-2-yl)amino)-5-fluor opyrimidin-4-yl)-4-fluoro-2-methyl-1H-benzo[d]imidazol-1-yl)butanoate **114** (0.5 g, 0.7 mmol) was dissolved in 4 mL of concentrated sulfuric acid at 0°C, and stirred for 30 minutes. The reaction was monitored by TLC (dichloromethane: methanol=5:1). After the reaction was completed, the reaction was stopped. The reaction solution was poured into crushed ice, and 10% sodium hydroxide solution was added to adjust pH = 8-9. The reaction solution was extracted with ethyl acetate (3 × 20 mL). The organic phases were combined, dried and concentrated to dryness by rotary evaporation to obtain the title compound as a brown solid (0.4 g, yield: 100%).

MS (ESI):580.6 $[M+1]^+$.

Step 13

3-(6-(2-((4-Amino-5-((4-ethylpiperazin-1-yl)methyl)pyridin-2-yl)amino)-5-fluoropyrim idin-4-yl)-4-fluoro-2-methyl-1H-benzo[d]imidazol-1-yl)butanoic acid **116**

Methyl 3-(6-(2-((4-amino-5-((4-ethylpiperazin-1-yl)methyl)pyridin-2-yl)amino)-5-fluoropyrimi din-4-yl)-4-fluoro-2-methyl-1H-benzo[d]imidazol-1-yl)butanoate **115** (0.4 g, 6.9 mmol) was dissolved in 5 mL methanol and 5 mL of 10% sodium hydroxide solution. The reaction solution was stirred at room temperature overnight. The reaction was monitored by TLC (dichloromethane: methanol=5:1). After the reaction was completed, the reaction was stopped. Concentrated hydrochloric acid was added to adjust pH = 4-5. The reaction solution was concentrated to dryness by rotary evaporation and dried. 50 mL of ethanol was added, and the solution was stirred for 5 hours, filtered, and washed. The filtrate was concentrated to dryness by rotary evaporation to obtain the title compound as a yellow solid (0.8 g, yield: 100%).

MS (ESI):566.6 $[M+1]^+$.

Step 14

$4^5$-((4-Ethylpiperazin-1-yl)methyl)-$1^4$,$2^5$-difluoro-$1^2$,8-dimethyl-$1^1$H-3,5-diaza-1(6,1)-b enzo[d]imidazola-2(4,2)-pyrimidina-4(2,4)-pyridinacyclooctaphan-6-one **1**

3-(6-(2-((4-Amino-5-((4-ethylpiperazin-1-yl)methyl)pyridin-2-yl)amino)-5-fluorop yrimidin-4-yl)-4-fluoro-2-methyl-1H-benzo[d]imidazol-1-yl)butanoic acid **116** (0.8 g, 0.7 mmol) and triethylamine (212 mg, 2.1 mmol) were dissolved in 50 mL of dichloromethane, followed by the addition of HATU (323 mg, 0.85 mmol). The reaction solution was stirred at room temperature overnight. The reaction was monitored by TLC (DCM:MeOH = 5:1). After the reaction was completed, the reaction was stopped. Water was added, and the reaction solution was extracted with dichloromethane (3 × 20 mL). The organic phases were combined, dried and concentrated to dryness by rotary evaporation to obtain the crude product. The crude product was purified by preparative silica gel plate (DCM:MeOH = 5:1) to obtain the title compound as a white solid (32 mg).

MS (ESI):548.6 $[M+1]^+$.

$^1H$ NMR (400 MHz, $CDCl_3$)) $\delta$ 10.41 (s, 1H), 10.21 (s, 1H), 9.73 (s, 1H), 8.89 - 8.47 (m, 2H), 8.12 (s, 1H), 7.59 (d, *J* = 11.8 Hz, 1H), 5.23 (s, 1H), 4.02 (m, 1H), 3.65 - 3.35 (m, 2H), 3.10 - 2.55 (m, 9H), 2.47 - 2.08 (m, 5H), 1.79 (d, *J* = 6.4 Hz, 3H), 1.03 (t, *J*= 6.5 Hz, 3H).

Example 2

(R)-$4^5$-((4-Ethylpiperazin-1-yl)methyl)-$1^4$,$2^5$-difluoro-$1^2$,8-dimethyl-$1^1$H-3,5-diaza-1(6, 1)-benzo[*d*]imidazola-2(4,2)-pyrimidina-4(2,4)-pyridinacyclooctaphan-6-one 2

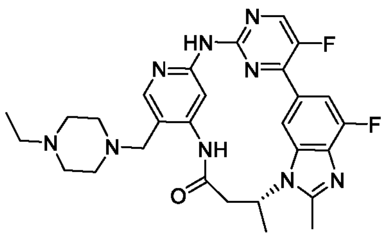

**2**

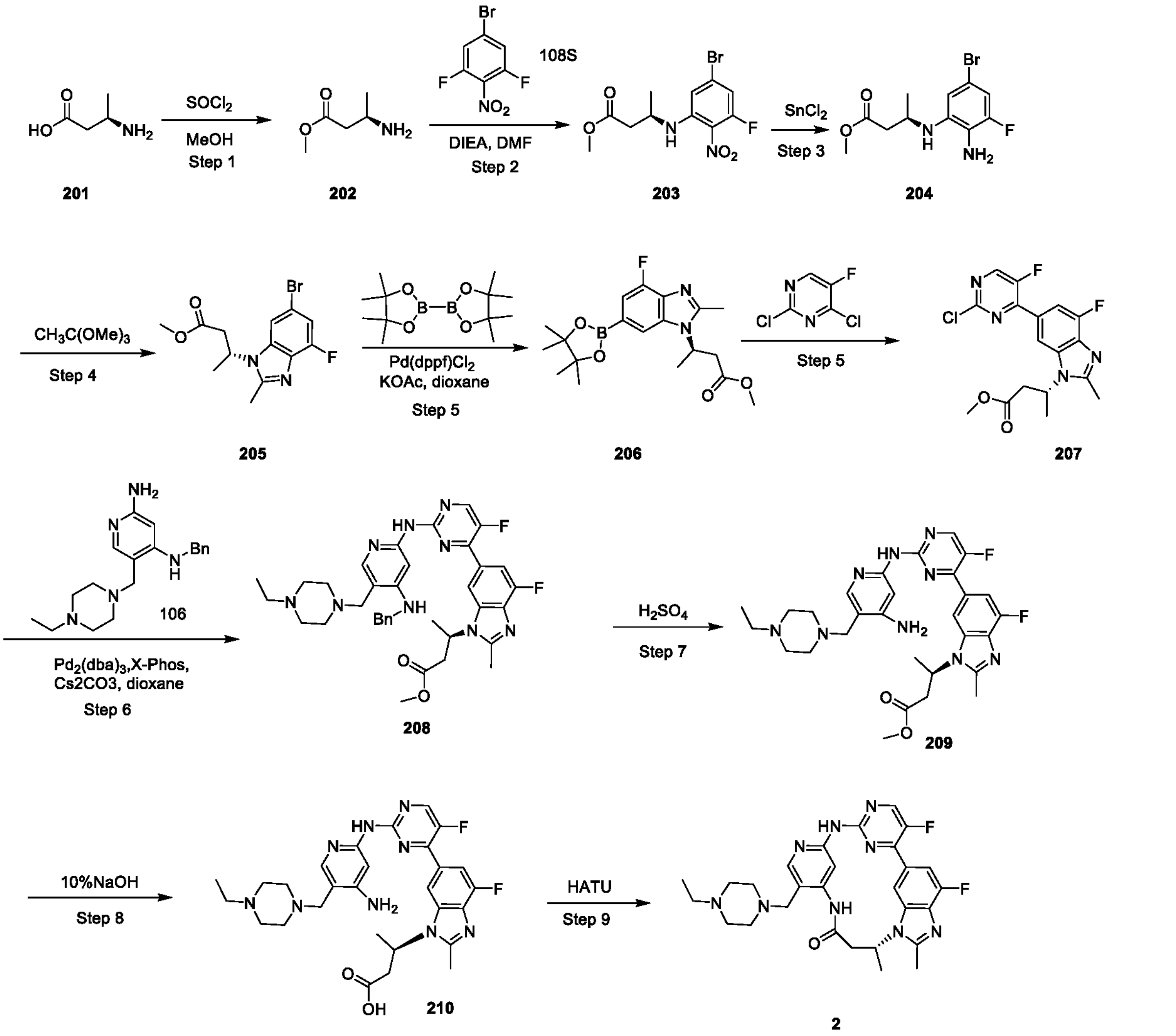


Step 1

Methyl (R)-3-aminobutanoate **202**

Thionyl chloride (5.1 g, 43 mmol) was added dropwise to 40 mL of methanol, and the solution was stirred for 1 hour at 0°C. (R)-3-Aminobutanoic acid **201** (3 g, 29 mmol) was added to the reaction system, which was then stirred at room temperature for 5 hours. The reaction was monitored by TLC (dichloromethane: methanol = 5:1). After the reaction was completed, the reaction solution was concentrated to dryness by rotary evaporation. Methanol was added, and then the solution was concentrated to dryness, and this process was repeated twice. The title compound was obtained as a colorless oil (4.0 g, yield: 100%). The resulting product was used directly in the next step.

Step 2

Methyl (R)-3-((5-bromo-3-fluoro-2-nitrophenyl)amino)butanoate **203**

1,3-Difluoro-5-bromo-2-nitrobenzene (6.8 g, 29 mmol) and methyl (R)-3-aminobutanoate **202** (4.0 g, 29 mmol) were dissolved in 50 mL of DMF, followed by the addition of DIEA (11.2 g, 87 mmol). The reaction solution was stirred at room temperature overnight. The reaction was monitored by TLC (petroleum ether: ethyl acetate = 5:1). After the reaction was completed, 100 mL of water was added, and the raction solution was extracted with ethyl acetate (3 × 25 mL). The organic phases were combined, washed with water, dried and concentrated to dryness by rotary evaporation to obtain the crude product. The crude product was purified by column chromatography (petroleum ether: ethyl acetate = 50:1) to obtain the title compound as a yellow solid (9 g, yield: 90.3%).

MS (ESI):335.2 $[M+1]^+$.

Step 3

Methyl (R)-3-((2-amino-5-bromo-3-fluorophenyl)amino)butanoate **204**

Methyl (R)-3-((5-bromo-3-fluoro-2-nitrophenyl)amino)butanoate **203** (9 g, 26.8 mmol) was dissolved in 50 mL of DMF, followed by the addition of stannous chloride (30.3 g, 34 mmol). The reaction solution was stirred at room temperature overnight. The reaction was monitored by TLC (petroleum ether: ethyl acetate = 5:1). After the reaction was completed, 100 mL of water was added, and the reaction solution was extracted with ethyl acetate (3 × 50 mL). The organic phases were combined, washed with water, dried and concentrated to dryness by rotary evaporation. The title compound was obtained as an orange oil (10 g, yield: 100%).

MS (ESI):306.2 $[M+1]^+$.

Step 4

Methyl (R)-3-(6-bromo-4-fluoro-2-methyl-1H-benzo[d]imidazol-1-yl)butanoate **205**

Methyl (R)-3-((2-amino-5-bromo-3-fluorophenyl)amino)butanoate **204** (10 g, 26.8 mmol) was dissolved in 45 mL of trimethyl orthoformate and 30 mL of acetic acid. The reaction solution was reacted at 90°C for 8 hours. The reaction was monitored by TLC (petroleum ether: ethyl acetate = 1:1). After the reaction was completed, the reaction solution was concentrated to dryness by rotary evaporation. Saturated sodium bicarbonate solution was added, and the reaction solution was extracted with ethyl acetate (3 × 25 mL). The organic phases were combined, washed with water, dried and concentrated to dryness by rotary evaporation to obtain the crude product. The crude product was dissolved in 20 mL of a solution of ethyl acetate and petroleum ether (1:10), stirred for one hour and filtered. The filter cake was washed with petroleum ether, and dried under reduced pressure to obtain the title compound as a white solid (6.2 g, yield: 69.7%).

MS (ESI):330.1 $[M+1]^+$.

Step 5

Methyl (R)-3-(6-(2-chloro-5-fluoropyrimidin-4-yl)-4-fluoro-2-methyl-1H-benzo[d]imidazol-1-y l)butanoate **207**

Methyl (R)-3-(6-bromo-4-fluoro-2-methyl-1H-benzo[d]imidazol-1-yl)butanoate **205** (5 g, 15.1 mmol), pinacol diborate (4.6 g, 18.2 mmol), potassium acetate (4.84 g, 45.3 mmol) and Pd(dppf)$Cl_2$ (0.64 g, 0.75 mmol) were dissolved in 120 mL of dioxane under a nitrogen atmosphere. The reaction solution was reacted at 100°C for 2 hours. The reaction was monitored by HPLC. After the reaction was completed, the intermediate 2,4-dichloro-5-fluoropyrimidine (3.2 g, 19.6 mmol), cesium carbonate (14.7 g, 45.3 mmol), Pd(dppf)$Cl_2$ (0.61 g, 0.7 mmol) and 12 mL of water were added to the reaction solution. The reaction solution was reacted at 110°C for 14 hours. The reaction was monitored by LC-MS. When the raw material no longer decreased, the reaction was stopped. The reaction solution was concentrated to dryness by rotary evaporation. Water was added, and the solution was extracted with ethyl acetate (3 × 40 mL). The organic phases were combined, dried and concentrated to dryness by rotary evaporation to obtain the crude product. The crude product was purified by column chromatography (petroleum ether: ethyl acetate = 1:1) to obtain the title compound as a yellow solid (3.1 g, yield: 53.6%).

MS(ESI):381.1$[M+1]^+$.

Step 6

Methyl (R)-3-(6-(2-((4-(benzylamino)-5-((4-ethylpiperazin-1-yl)methyl)pyridin-2-yl)amino)-5-fluoropyrimidin-4-yl)-4-fluoro-2-methyl-1H-benzo[d]imidazol-1-yl)butanoate **208**

Methyl (R)-3-(6-(2-chloro-5-fluoropyrimidin-4-yl)-4-fluoro-2-methyl-1H-benzo[d]imidazol-1-y l)butanoate **207** (1.1 g, 2.95 mmol), $N^4$-benzyl-5-((4-ethylpiperazin-1-yl)methyl)pyridine-2,4-diamine (0.8 g, 2.46 mmol), $Pd_2(dba)_3$ (112 mg, 0.12 mmol), X-phos (175 mg, 0.37 mmol) and potassium carbonate (1.02 g, 7.38 mmol) were suspended in 100 mL of dioxane under a nitrogen atmosphere. The reaction solution was reacted at 100°C for 12 hours. The reaction was monitored by TLC (dichloromethane: methanol=5:1). After the reaction was completed, the reaction was stopped. The reaction solution was concentrated to dryness by rotary evaporation. Water was added, and the solution was extracted with dichloromethane (3 × 30 mL). The organic phases were combined, dried and concentrated to dryness by rotary evaporation to obtain the crude product. The crude product was purified by column chromatography (dichloromethane: methanol=5:1) to obtain the title compound as a brown solid (0.4 g, yield: 20.3%).

MS (ESI):670.7 $[M+1]^+$.

Step 7

Methyl (R)-3-(6-(2-((4-amino-5-((4-ethylpiperazin-1-yl)methyl)pyridin-2-yl)amino)-5-fluoropy rimidin-4-yl)-4-fluoro-2-methyl-1H-benzo[d]imidazol-1-yl)butanoate **209**

Methyl (R)-3-(6-(2-((4-(benzylamino)-5-((4-ethylpiperazin-1-yl)methyl)pyridin-2-yl)amino)-5-fluoropyrimidin-4-yl)-4-fluoro-2-methyl-1H-benzo[d]imidazol-1-yl)butanoate **208** (0.4 g, 0.6 mmol) was dissolved in 4 mL of concentrated sulfuric acid at 0°C, and the solution was stirred for 30 minutes. The reaction was monitored by TLC (dichloromethane: methanol=5:1). After the reaction was completed, the reaction was stopped. The reaction solution was poured into crushed ice, and 10% sodium hydroxide solution was added to adjust pH = 8-9. The reaction solution was extracted with ethyl acetate (3 × 30 mL). The organic phases were combined, dried and concentrated to dryness by rotary evaporation to obtain the title compound as a brown solid (0.4 g, yield: 100%).

Step 8

(R)-3-(6-(2-((4-Amino-5-((4-ethylpiperazin-1-yl)methyl)pyridin-2-yl)amino)-5-fluorop yrimidin-4-yl)-4-fluoro-2-methyl-1H-benzo[d]imidazol-1-yl)butanoic acid **210**

Methyl (R)-3-(6-(2-((4-amino-5-((4-ethylpiperazin-1-yl)methyl)pyridin-2-yl)amino)-5-fluoropy rimidin-4-yl)-4-fluoro-2-methyl-1H-benzo[d]imidazol-1-yl)butanoate **209** (0.4 g, 6.9 mmol) was dissolved in 5 mL methanol and 5 mL of 10% sodium hydroxide solution. The reaction solution was stirred at room temperature overnight. The reaction was monitored by TLC (dichloromethane: methanol=5:1). After the reaction was completed, the reaction was stopped. Concentrated hydrochloric acid was added to adjust pH = 4-5. The reaction solution was concentrated to dryness by rotary evaporation and dried. 50 mL of ethanol was added, and the solution was stirred for 5 hours, filtered, and washed. The filtrate was concentrated to dryness by rotary evaporation to obtain the title compound as a yellow solid (0.5 g, yield: 100%).
MS(ESI):566.6$[M+1]^+$.

Step 9

(R)-$4^5$-((4-Ethylpiperazin-1-yl)methyl)-$1^4$,$2^5$-difluoro-$1^2$,8-dimethyl-$1^1$H-3,5-diaza-1(6, 1)-benzo[*d*]imidazola-2(4,2)-pyrimidina-4(2,4)-pyridinacyclooctaphan-6-one **2**

(R)-3-(6-(2-((4-Amino-5-((4-ethylpiperazin-l-yl)methyl)pyridin-2-yl)amino)-5-flu oropyrimidin-4-yl)-4-fluoro-2-methyl-1H-benzo[d]imidazol-1-yl)butanoic acid **210** (0.5 g, 0.69 mmol) and triethylamine (212 mg, 2.1 mmol) were dissolved in 50 mL of dichloromethane, followed by the addition of HATU (323 mg, 0.85 mmol). The reaction solution was stirred at room temperature overnight. The reaction was monitored by TLC (dichloromethane: methanol=5: 1). After the reaction was completed, the reaction was stopped. Water was added, and the reaction solution was extracted with dichloromethane (3 × 30 mL). The organic phases were combined, dried and concentrated to dryness by rotary evaporation to obtain the crude product. The crude product was purified by preparative silica gel plate (dichloromethane: methanol=5: 1) to obtain the title compound as a white solid (24 mg, yield: 7.4%).
MS(ESI):548.6$[M+ 1]^+$.
$^1$H NMR (400 MHz, DMSO) $\delta$ 10.41 (s, 1H), 10.21 (s, 1H), 9.73 (s, 1H), 8.89 - 8.47 (m, 2H), 8.12 (s, 1H), 7.59 (d, *J* = 11.8 Hz, 1H), 5.23 (s, 1H), 3.85 (d, *J* = 13.2 Hz, 1H), 3.65 - 3.35 (m, 2H), 3.10 - 2.55 (m, 9H), 2.47 - 2.08 (m, 5H), 1.79 (d, *J* = 6.4 Hz, 3H), 1.03 (t, *J* = 6.5 Hz, 3H).

Example 3

(S)-$4^5$-((4-Ethylpiperazin-1-yl)methyl)-$1^4$,$2^5$-difluoro-$1^2$,8-dimethyl-$1^1$H-3,5-diaza-1(6, 1)-benzo[*d*]imidazola-2(4,2)-pyrimidina-4(2,4)-pyridinacyclooctaphan-6-one **3**

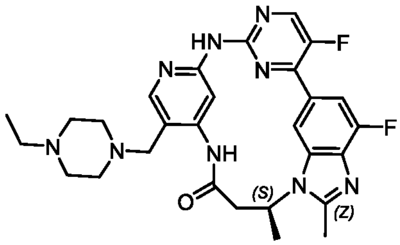

**3**

In accordance with the method described in Example 2, methyl (S)-3-aminobutyrate was used as the starting material, accordingly, (S)-4$^5$-((4-Ethylpiperazin-1-yl)methyl)-1$^4$,2$^5$-difluoro-1$^2$,8-dimethyl-1$^1$H-3,5-diaza-1(6, 1)-benzo[*d*]imidazola-2(4,2)-pyrimidina-4(2,4)-pyridinacyclooctaphan-6-one **3** was prepared as a white solid.

MS(ESI):548.6[M+1]$^+$.

$^1$H NMR (400 MHz, DMSO) δ 10.41 (s, 1H), 10.21 (s, 1H), 9.73 (s, 1H), 8.89 - 8.47 (m, 2H), 8.12 (s, 1H), 7.59 (d, *J* = 11.8 Hz, 1H), 5.23 (s, 1H), 3.85 (d, *J* = 13.2 Hz, 1H), 3.65 - 3.35 (m, 2H), 3.10 - 2.55 (m, 9H), 2.47 - 2.08 (m, 5H), 1.79 (d, *J* = 6.4 Hz, 3H), 1.03 (t, *J* = 6.5 Hz, 3H).

Example 4

4$^5$-((4-Ethylpiperazin-1-yl)methyl)-1$^4$2$^5$-difluoro-1$^2$-methyl-1$^1$H-3,5-diaza-1(6,1)-benzo [d]imidazola-2(4,2)-pyrimidina-4(2,4)-pyridinacyclooctaphan-6-one **4**

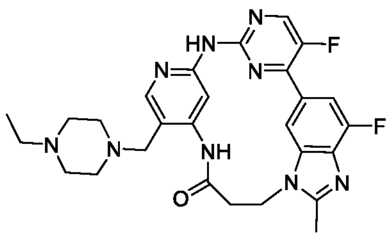

**4**

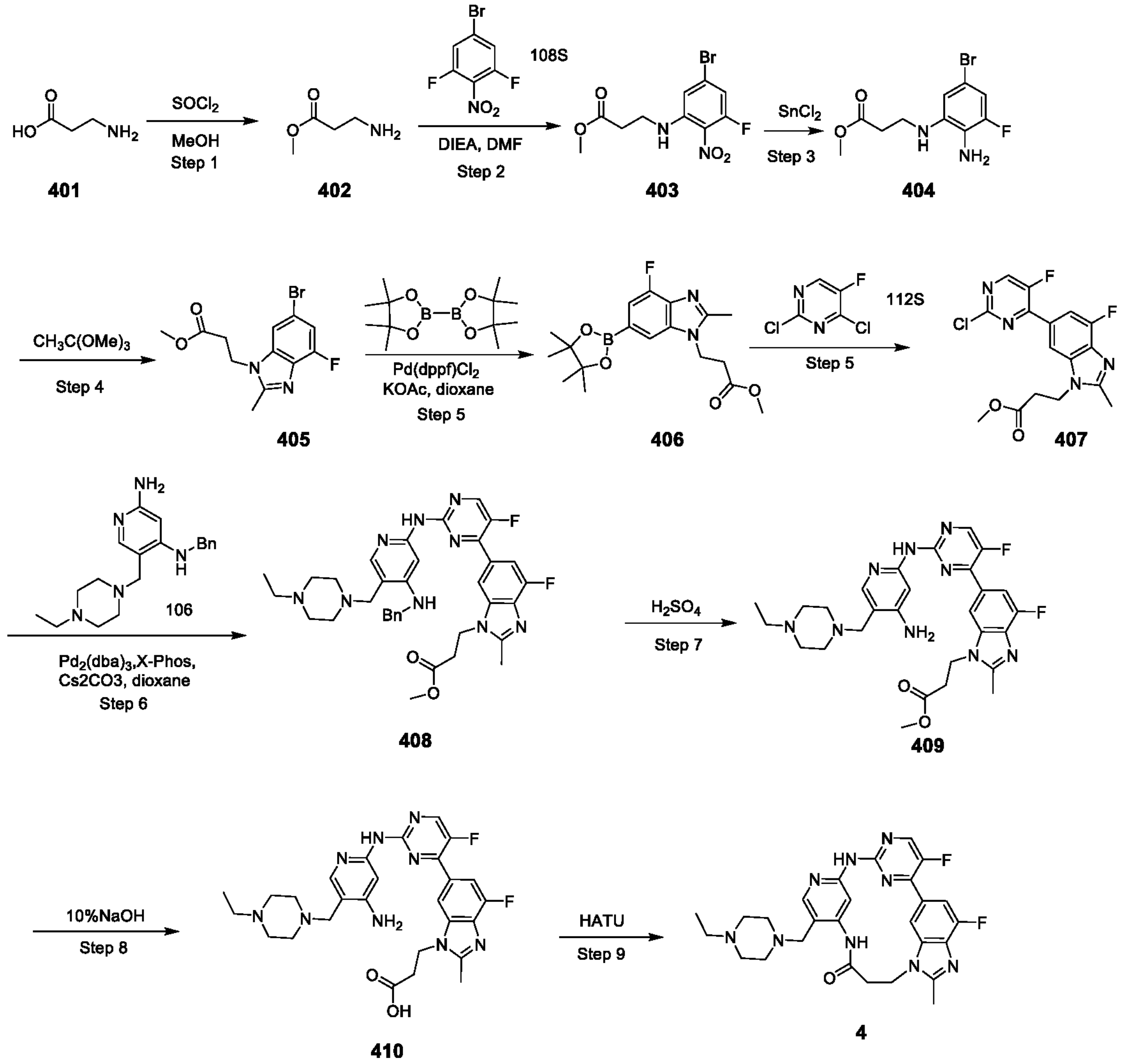


Step 1

Methyl aminopropanoate **402**

Thionyl chloride (10 g, 84 mmol) was added dropwise to 40 mL of methanol and stirred for 1 hour at 0°C. Aminopropanoic acid (5 g, 56 mmol) was added to the reaction system and stirred at room temperature for 5 hours. The reaction was monitored by TLC (DCM:MeOH = 5:1). After the reaction was completed, the reaction solution was concentrated to dryness by rotary evaporation. MeOH was added, and then the solution was concentrated to dryness, and this process was repeated twice. The title compound was obtained as a colorless oil (6 g, yield: 100%). The resulting product was used directly in the next step.

Step 2

Methyl 3-((5-bromo-3-fluoro-2-nitrophenyl)amino)propanoate **403**

1,3-Difluoro-5-bromo-2-nitrobenzene (13.3 g, 56 mmol) and methyl 3-aminopropanoate **402** (6 g, 56 mmol) were dissolved in 100 mL of DMF, followed by the addition of DIEA (21.6 g, 168 mmol). The reaction solution was stirred at room temperature overnight. The reaction was monitored by TLC (PE:EA = 5:1). After the reaction was completed, 150 mL of water was added, and the reaction solution was extracted with 150 mL of ethyl acetate twice. The organic phases were combined, washed with water, dried and concentrated to dryness by rotary evaporation to obtain the crude product. The crude product was purified by column chromatography (PE:EA = 50:1) to obtain the title compound as a orange solid (14 g, yield: 77.8%).
MS (ESI): 322.1 $[M+1]^+$.

Step 3

Methyl 3-((2-amino-5-bromo-3-fluorophenyl)amino)propanoate **404**

Methyl 3-((5-bromo-3-fluoro-2-nitrophenyl)amino)propanoate **403** (14 g, 43.6 mmol) was dissolved in 150 mL of DMF, followed by the addition of stannous chloride (49 g, 218 mmol). The reaction solution was stirred at room temperature overnight. The reaction was monitored by TLC (petroleum ether: ethyl acetate = 5:1). After the reaction was completed, 500 mL of water was added, and the reaction solution was extracted with ethyl acetate (50 mL × 3). The organic phases were combined, washed with water, dried and concentrated to dryness by rotary evaporation. The title compound was obtained as a pale yellow oil (12 g, yield: 100%).
MS (ESI): 292.2 $[M+1]^+$.

Step 4

Methyl 3-(6-bromo-4-fluoro-2-methyl-1H-benzo[d]imidazol-1-yl)propanoate **405**

Methyl 3-((2-amino-5-bromo-3-fluorophenyl)amino)propanoate **404** (12 g, 41 mmol) was dissolved in 90 mL of trimethyl orthoacetate and 54 mL of acetic acid. The reaction solution was reacted at 90°C for 8 hours. The reaction was monitored by TLC (petroleum ether: ethyl acetate = 1:1). After the reaction was completed, the reaction solution was concentrated to dryness by rotary evaporation. Saturated sodium bicarbonate solution (about 100 mL) was added, and the reaction solution was extracted with ethyl acetate (50 ml×3). The organic phases were combined, washed with water, dried and concentrated to dryness by rotary evaporation to obtain the crude product. The crude product was dissolved in 10 mL of a solution of ethyl acetate and petroleum ether (1:10), and the solution was stirred for one hour, filtered, washed with petroleum ether, and dried under reduced pressure to obtain the title compound as a white solid (6.8 g, yield: 52.3%).
MS (ESI): 316.1 $[M+1]^+$.

Step 5

Methyl 3-(6-(2-chloro-5-fluoropyrimidin-4-yl)-4-fluoro-2-methyl-1H-benzo[d]imidazol-1-yl)pr opanoate **407**

Methyl 3-(6-bromo-4-fluoro-2-methyl-1H-benzo[d]imidazol-1-yl)propanoate **405** (5 g, 15.9 mmol), pinacol diborate (4.8 g, 19.1 mmol), potassium acetate (4.7 g, 47.7 mmol) and $Pd(dppf)Cl_2$ (650 mg, 0.8 mmol) were dissolved in 100 mL of dioxane under a nitrogen atmosphere. The reaction solution was reacted at 100°C for 2 hours. The reaction was monitored by HPLC. After the reaction was completed, the intermediate 2,4-dichloro-5-fluoropyrimidine (3.4 g, 20.4 mmol), cesium carbonate (15.5 g, 47.7 mmol), $Pd(dppf)Cl_2$ (650 mg, 0.8 mmol) and 15 mL of water were added to the reaction solution. The reaction solution was reacted at 110°C for 14 hours. The reaction was monitored by LC-MS. When the raw material no longer decreased, the reaction was stopped. The reaction solution was concentrated to dryness by rotary evaporation. Water (about 100 ml) was added, and the solution was extracted with ethyl acetate (50 ml × 3). The organic phases were combined, dried and concentrated to dryness by rotary evaporation to obtain the crude product. The crude product was purified by silica gel column chromatography (petroleum ether: ethyl acetate = 1:1) to obtain the title compound as a brown solid (2.8 g, yield: 48.2%).
MS (ESI): 367.1 $[M+1]^+$.

Step 6

Methyl 3-(6-(2-((4-(benzylamino)-5-((4-ethylpiperazin-1-yl)methyl)pyridin-2-yl)amino)-5-fluor opyrimidin-4-yl)-4-fluoro-2-methyl-1H-benzo[d]imidazol-1-yl)propanoate **408**

Methyl 3-(6-(2-chloro-5-fluoropyrimidin-4-yl)-4-fluoro-2-methyl-1H-benzo[d]imidazol-1-yl)pr opanoate **407** (2.8 g, 7.6 mmol), $N^4$-benzyl-5-((4-ethylpiperazin-1-yl)methyl)pyridine-2,4-diamine (2 g, 6.3 mmol), $Pd_2(dba)_3$ (580 mg, 0.63 mmol), X-phos (900 mg, 1.89 mmol) and potassium carbonate (2.6 g, 18.9 mmol) were suspended in 70 mL of dioxane under a nitrogen atmosphere. The reaction solution was reacted at 100°C for 12 hours. The reaction was monitored by TLC (DCM:MeOH =5:1). After the reaction was completed, the reaction was stopped. The reaction solution was concentrated to dryness by rotary evaporation. Water (about 70 ml) was added, and the solution was extracted with ethyl acetate (50 ml × 3). The organic phases were combined, dried and concentrated to dryness by rotary evaporation to obtain the crude product. The crude product was purified by silica gel column chromatography (DCM:MeOH = 5:1) to obtain the title compound as a brown oil (1.5 g, yield: 30.9%).

MS (ESI): 656.7 $[M+1]^+$.

Step 7

Methyl 3-(6-(2-((4-amino-5-((4-ethylpiperazin-1-yl)methyl)pyridin-2-yl)amino)-5-fluoropyrimi din-4-yl)-4-fluoro-2-methyl-1H-benzo[d]imidazol-1-yl)propanoate **409**

Methyl 3-(6-(2-((4-(benzylamino)-5-((4-ethylpiperazin-1-yl)methyl)pyridin-2-yl)amino)-5-fluor opyrimidin-4-yl)-4-fluoro-2-methyl-1H-benzo[d]imidazol-1-yl)propanoate **408** (1.5 g, 2.1 mmol) was dissolved in 4 mL of concentrated sulfuric acid at 0°C, and the soultion was stirred for 30 minutes. The reaction was monitored by TLC (DCM:MeOH = 5:1). After the reaction was completed, the reaction was stopped. The reaction solution was poured into crushed ice (about 100 g), and 10% sodium hydroxide solution was added to adjust pH = 8-9. The reaction solution was extracted with ethyl acetate (50 ml × 3). The organic phases were combined, dried and concentrated to dryness by rotary evaporation to obtain the title compound as a brown solid (0.9 g, yield: 63.6%).

MS (ESI): 566.6 $[M+1]^+$.

Step 8

3-(6-(2-((4-Amino-5-((4-ethylpiperazin-1-yl)methyl)pyridin-2-yl)amino)-5-fluoropyrim idin-4-yl)-4-fluoro-2-methyl-1H-benzo[d]imidazol-1-yl)propanoic acid **410**

Methyl 3-(6-(2-((4-amino-5-((4-ethylpiperazin-1-yl)methyl)pyridin-2-yl)amino)-5-fluoropyrimi din-4-yl)-4-fluoro-2-methyl-1H-benzo[d]imidazol-1-yl)propanoate **409** (0.9 g, 1.6 mmol) was dissolved in 5 mL methanol and 5 mL of 10% sodium hydroxide solution. The reaction solution was stirred at room temperature overnight. The reaction was monitored by TLC (DCM:MeOH = 5:1). After the reaction was completed, the reaction was stopped. Concentrated hydrochloric acid was added to adjust pH = 4-5. The reaction solution was concentrated to dryness by rotary evaporation and dried. 50 mL of ethanol was added, and the solution was stirred for 5 hours, filtered, and washed. The filtrate was concentrated to dryness by rotary evaporation to obtain the title compound as an off-white solid (1.6 g, yield: 100%).

MS (ESI): 552.6 $[M+1]^+$.

Step 9

$4^5$-((4-Ethylpiperazin-1-yl)methyl)-$1^4$,$2^5$-difluoro-$1^2$-methyl-$1^1$H-3,5-diaza-1(6,1)-benzo [d]imidazola-2(4,2)-pyrimidina-4(2,4)-pyridinacyclooctaphan-6-one **4**

3-(6-(2-((4-Amino-5-((4-ethylpiperazin-1-yl)methyl)pyridin-2-yl)amino)-5-fluorop yrimidin-4-yl)-4-fluoro-2-methyl-1H-benzo[d]imidazol-1-yl)propanoic acid **410** (1.6 g, 0.9 mmol) and triethylamine (270 mg, 2.7 mmol) were dissolved in 50 mL of dichloromethane, followed by the addition of HATU (510 mg, 1.3 mmol). The reaction solution was stirred at room temperature overnight. The reaction was monitored by TLC (DCM:MeOH =5:1). After the reaction was completed, the reaction was stopped. Water (about 50 ml) was added, and the reaction solution was separated into two phases. The aqueous phase was extracted with ethyl acetate (50 ml × 3). The organic phase was dried and concentrated to dryness by rotary evaporation to obtain the crude product. The crude product was purified by preparative silica gel plate (DCM:MeOH = 5:1) to obtain the title compound as a white solid (35 mg).

MS (ESI): 534.6 [M+1]+.

$^1$H NMR (400 MHz, DMSO-$d_6$) δ 10.68 (s, 1H), 10.11 (s, 1H), 9.52 (s, 1H), 8.67 (d, *J* = 4.1 Hz, 1H), 8.39 (s, 1H), 8.08 (s, 1H), 7.58 (d, *J* = 11.8 Hz, 1H), 4.71 (s, 2H), 3.97-3.41 (m, 2H), 3.03 - 2.60 (m, 9H), 2.48 - 1.93 (m, 6H), 1.02 (s, 3H).

Example 5

$4^5$-((4-Ethylpiperazin-1-yl)methyl)-$1^4$,$2^5$-difluoro-$1^2$,8-dimethyl-$1^1$H-3,5-diaza-1(6,1)-b enzo[d]imidazola-2(4,2)-pyrimidina-4(2,4)-pyridinacyclooctaphane 5

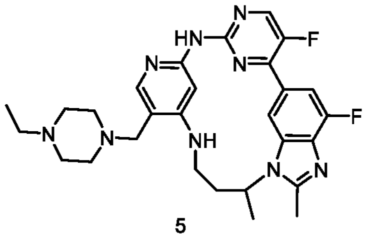

5

$LiAlH_4$ (10 mg, 0.26 mmol) was added to a solution of $4^5$-((4-ethylpiperazin-1-yl)methyl)-$1^4$,$2^5$-difluoro-$l^2$,8-dimethyl-$l^1$H-3,5-diaza-1(6,1)-be nzo[d]imidazola-2(4,2)-pyrimidina-4(2,4)-pyridinacyclooctaphan-6-one **1** (100 mg, 0.182 mmol) in THF (5 mL) at 0°C. The reaction solution was stirred at room temperature overnight. The reaction was monitored by TLC (DCM:MeOH = 5:1). After the reaction was completed, the reaction was stopped. Saturated aqueous ammonium chloride solution (about 10 ml) was added at 0°C, and the reaction solution was separated into two phases. The aqueous phase was extracted with ethyl acetate (10 ml × 3). The organic phase was dried and concentrated to dryness by rotary evaporation to obtain the crude product. The crude product was purified by preparative silica gel plate (DCM:MeOH = 5:1) to obtain the title compound as a white solid (60 mg).

MS (ESI): 534.2 [M+1]+.

$^1H$ NMR (400 MHz, $CDCl_3$)) $\delta$ 10.41 (s, 1H), 10.21 (s, 1H), 9.73 (s, 1H), 8.89 - 8.47 (m, 2H), 8.12 (s, 1H), 7.59 (d, *J* = 11.8 Hz, 1H), 5.23 (s, 1H), 3.85 (d, *J* = 13.2 Hz, 1H), 3.32 - 3.20 (m, 2H), 3.10 - 2.55 (m, 9H), 2.47 - 2.08 (m, 5H), 2.00 - 1.84 (m, 2H), 1.79 (d, *J*= 6.4 Hz, 3H), 1.03 (t, *J*= 6.5 Hz, 3H).

Example 7

$4^5$-((4-Ethylpiperazin-1-yl)methyl)-$2^5$-fluoro-8-methyl-$1^1$H-3,5-diaza-1(6,1)-indola-2(4, 2)-pyrimidina-4(2,4)-pyridinacyclooctaphan-6-one 7

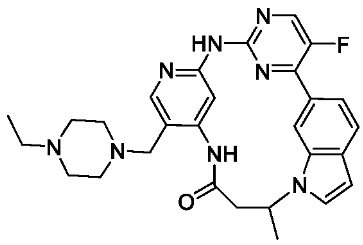

7

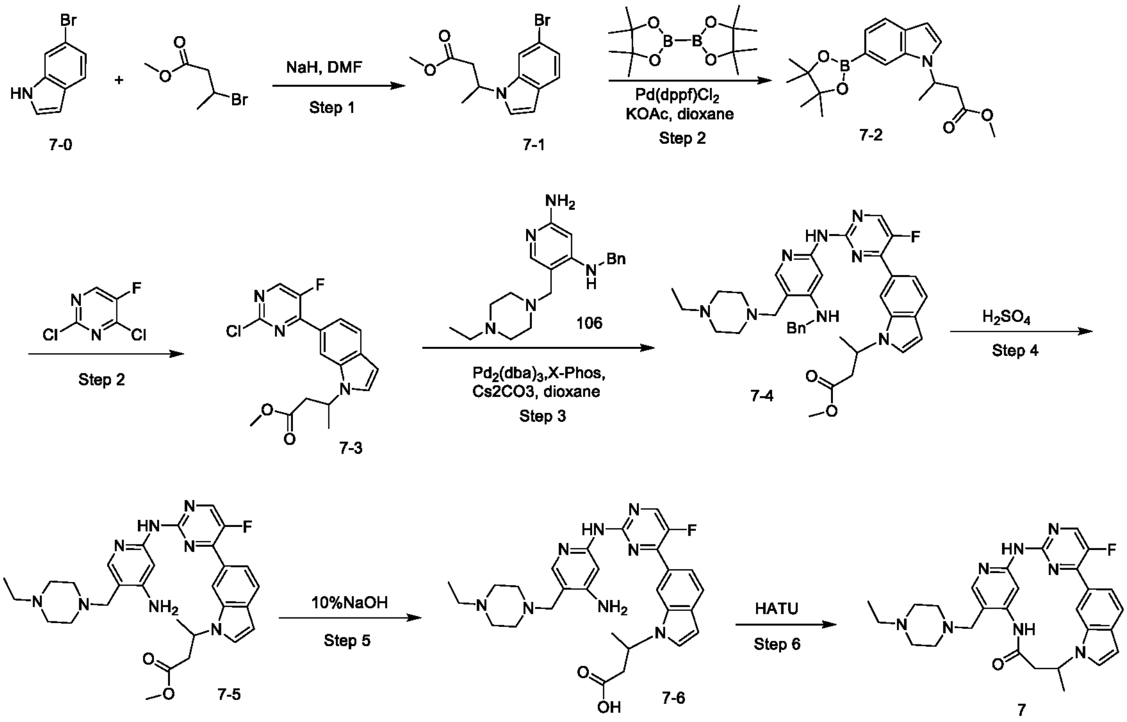


Step 1

Methyl 3-(6-bromo-1H-indol-1-yl)butanoate **7-1**

6-Bromoindole (1.0 g, 5.1 mmol), methyl 3-bromobutanoate (1.38 g, 7.65 mmol) and sodium hydroxide (0.43 g, 7.65 mmol) were added to N,N-dimethylformamide (DMF, 10 mL). The reaction solution was reacted under a nitrogen atmosphere at 70°C for 10 hours. Water (50 mL)/ethyl acetate (50 mL) were added to the reaction solution and stirred for extraction. The organic phase was dried, filtered and concentrated. The resulting residue was purified by silica gel column chromatography (eluent: ethyl acetate/petroleum ether: 1/30-1/10) to obtain compound **7-1** as a white solid (1.2 g, yield: 80%). MS (ESI):296.1 $[M+1]^+$.

Step 2

Methyl 3-(6-(2-chloro-5-fluoropyrimidin-4-yl)-1H-indol-1-yl)butanoate **7-3**

Methyl 3-(6-bromo-1H-indol-1-yl)butanoate **7-1** (2.4 g, 8 mmol), pinacol diborate (2.3 g, 9.1 mmol), potassium acetate (2.4 g, 24 mmol) and Pd(dppf)$Cl_2$ (326 mg, 0.4 mmol) were dissolved in 70 mL of dioxane under a nitrogen atmosphere. The reaction solution was reacted at 100°C for 2 hours. The reaction was monitored by HPLC. After the reaction was completed, the intermediate 2,4-dichloro-5-fluoropyrimidine (1.7 g, 10.4 mmol), cesium carbonate (7.8 g, 24 mmol), Pd(dppf)$Cl_2$ (326 mg, 0.4 mmol) and 7 mL of water were added to the reaction solution. The reaction solution was reacted at 110°C for 14 hours. The reaction was monitored by LC-MS. When the raw material no longer decreased, the reaction was stopped. The reaction solution was concentrated to dryness by rotary evaporation. Water was added, and the solution was extracted with ethyl acetate (3 × 25 mL). The organic phases were combined, dried and concentrated to dryness by rotary evaporation to obtain the crude product. The crude product was purified by column chromatography (petroleum ether: ethyl acetate = 1:1) to obtain the title compound as a yellow solid (1 g, yield: 32.5%).

MS (ESI):348.1 $[M+1]^+$.

Step 3

Methyl 3-(6-(2-((4-(benzylamino)-5-((4-ethylpiperazin-1-yl)methyl)pyridin-2-yl)amino)-5-fluor opyrimidin-4-yl)-1H-indol-1-yl)butanoate **7-4**

Methyl 3-(6-(2-chloro-5-fluoropyrimidin-4-yl)-1H-indol-1-yl)butanoate **7-3** (0.63 g, 1.8 mmol), $N^4$-benzyl-5-((4-ethylpiperazin-1-yl)methyl)pyridine-2,4-diamine (0.72 g, 2.2 mmol), $Pd_2(dba)_3$ (82 mg, 0.09 mmol), 2-dicyclohexylphosphorus-2,4,6-triisopropylbiphenyl (X-phos) (128 mg, 0.27 mmol) and potassium carbonate (0.745 g, 5.4 mmol) were suspended in 70 mL of dioxane under a nitrogen atmosphere. The reaction solution was reacted at 100°C for 12 hours. The reaction was monitored by TLC (dichloromethane: methanol=5:1). After the reaction was completed, the reaction was stopped. The reaction solution was concentrated to dryness by rotary evaporation. Water was added, and the solution was extracted with dichloromethane (3 × 25 mL). The organic phases were combined, dried and concentrated to dryness by rotary evaporation to obtain the crude product. The crude product was purified by column chromatography (dichloromethane: methanol=5:1) to obtain the title compound as a brown solid (0.45 g, yield: 40%).

MS (ESI): 637.1 $[M+1]^+$.

Step 4

Methyl 3-(6-(2-((4-amino-5-((4-ethylpiperazin-1-yl)methyl)pyridin-2-yl)amino)-5-fluoropyrimi din-4-yl)-1H-indol-1-yl)butanoate **7-5**

Methyl 3-(6-(2-((4-(benzylamino)-5-((4-ethylpiperazin-1-yl)methyl)pyridin-2-yl)amino)-5-fluor opyrimidin-4-yl)-1H-indol-1-yl)butanoate **7-4** (0.45 g, 0.7 mmol) was dissolved in 4 mL of concentrated sulfuric acid at 0°C, and the solution was stirred for 30 minutes. The reaction was monitored by TLC (dichloromethane: methanol=5:1). After the reaction was completed, the reaction was stopped. The reaction solution was poured into crushed ice, and 10% sodium hydroxide solution was added to adjust pH = 8-9. The reaction solution was extracted with ethyl acetate (3 × 20 mL). The organic phases were combined, dried and concentrated to dryness by rotary evaporation to obtain the title compound as a brown solid (0.4 g, yield: 100%).

MS (ESI):547.1 $[M+1]^+$.

Step 5

3-(6-(2-((4-Amino-5-((4-ethylpiperazin-1-yl)methyl)pyridin-2-yl)amino)-5-fluoropyrim idin-4-yl)-1H-indol-1-yl)butanoic acid **7-6**

Methyl 3-(6-(2-((4-amino-5-((4-ethylpiperazin-1-yl)methyl)pyridin-2-yl)amino)-5-fluoropyrimi din-4-yl)-1H-indol-1-yl)butanoate **7-5** (0.38 g, 6.9 mmol) was dissolved in 5 mL methanol and 5 mL of 10% sodium hydroxide solution. The reaction solution was stirred at room temperature overnight. The reaction was monitored by TLC (dichloromethane: methanol=5:1). After the reaction was completed, the reaction was stopped. Concentrated hydrochloric acid was added to adjust pH = 4-5. The reaction solution was concentrated to dryness by rotary evaporation and dried. 50 mL of ethanol was added, and the solution stirred for 5 hours, filtered, and washed. The filtrate was concentrated to dryness by rotary evaporation to obtain the title compound as a yellow solid (0.38 g, yield: 100%).

MS (ESI):551.1 $[M+1]^+$.

Step 6

$4^5$-((4-Ethylpiperazin-1-yl)methyl)-$2^5$-difluoro-8-dimethyl-$1^1$H-3,5-diaza-1(6,1)-indola-2(4,2)-pyrimidina-4(2,4)-pyridinacyclooctaphan-6-one 7

3-(6-(2-((4-Amino-5-((4-ethylpiperazin-1-yl)methyl)pyridin-2-yl)amino)-5-fluorop yrimidin-4-yl)-1H-indol-1-yl)butanoic acid **7-6** (0.38 g, 0.7 mmol) and triethylamine (212 mg, 2.1 mmol) were dissolved in 50 mL of dichloromethane, followed by the addition of HATU (323 mg, 0.85 mmol). The reaction solution was stirred at room temperature overnight. The reaction was monitored by TLC (DCM:MeOH =5:1). After the reaction was completed, the reaction was stopped. Water was added, and the reaction solution was extracted with dichloromethane (3 × 20 mL). The organic phases were combined, dried and concentrated to dryness by rotary evaporation to obtain the crude product. The crude product was purified by preparative silica gel plate (DCM:MeOH = 5:1) to obtain the title compound 7 as a white solid (120 mg).

MS (ESI):515.2 $[M+1]^+$.

$^1$H NMR (400 MHz, $CDCl_3$)) δ 10.23 (s, 1H), 10.01 (s, 1H), 9.21 (s, 1H), 8.45 - 8.20 (m, 2H), 8.12 (d, J = 11.8 Hz, 1H), 7.82(s, 1H), 7.59 (d, *J* = 11.8 Hz, 1H), 7.33 (d, J = 12.1 Hz, 1H), 6.41 (d, J = 12.1 Hz, 1H), 5.23 (s, 1H), 4.5 (br, 1H), 4.06 (m, 1H), 3.65 (s, 2H), 2.84 - 2.62 (m, 2H), 2.47 - 2.08 (m, 8H), 1.68 (d, *J* = 6.2 Hz, 3H), 1.01 (t, *J*= 6.5 Hz, 3H).

Example 8

$4^5$-((4-Ethylpiperazin-1-yl)methyl)-$1^4$,$2^5$-difluoro-$1^2$,8,8-trimethyl-$1^1$H-3,5-diaza-1(6,1)-benzo[d]imidazola-2(4,2)-pyrimidina-4(2,4)-pyridinacyclooctaphan-6-one **8**

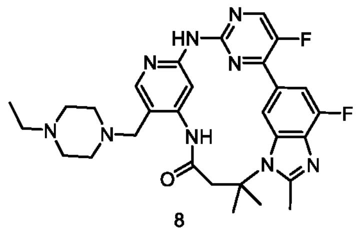

8

In accordance with the method described in Example 1, 3-aminobutyric acid **107** in Step 6 was replaced with 3-amino-3-methylbutyric acid, accordingly, $4^5$-((4-Ethylpiperazin-1-yl)methyl)-$1^4$,$2^5$-difluoro-$1^2$,8,8-trimethyl-$1^1$H-3,5-diaza-1(6,1)-benzo[d]imidazola-2(4,2)-pyrimidina-4(2,4)-pyridinacyclooctaphan-6-one **8** was prepared as a white solid.

MS (ESI):562.2 $[M+1]^+$.

$^1$H NMR (400 MHz, $CDCl_3$)) $\delta$ 10.31 (s, 1H), 10.01 (s, 1H), 9.73 (s, 1H), 8.89 - 8.47 (m, 2H), 8.12 (s, 1H), 7.62 (d, *J*= 11.8 Hz, 1H), 3.75 (s, 2H), 3.35 (q, J =6.5 Hz, 2H), 2.64 (s, 2H), 2.51 (s, 3H), 2.47 - 2.35 (m, 8H), 1.43 (s, 6H), 1.03 (t, *J* = 6.5 Hz, 3H).

Example 9

5'-((4-Ethylpiperazin-1-yl)methyl)-4',5'-difluoro-2'-methylspiro[cyclopropane-1,8'-3,5-d iaza-1(6,1)-benzo[d]imidazola-2(4,2)-pyrimidina-4(2,4)-pyridinacyclooctaphan]-6'-one

**9**

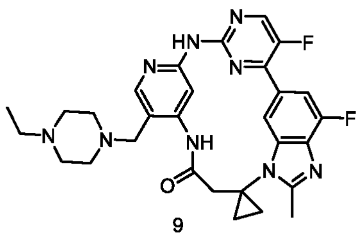

9

In accordance with the method described in Example 1, 3-aminobutyric acid **107** in Step 6 was replaced with 2-(1-aminocyclopropyl)acetic acid, accordingly, 5'-((4-ethylpiperazin-1-yl)methyl)-4',5'-difluoro-2'-methylspiro[cyclopropane-1,8'-3,5-di aza-1(6,1)-benzo[d]imidazola-2(4,2)-pyrimidina-4(2,4)-pyridinacyclooctaphan]-6'-one 9 was prepared as a white solid.

MS (ESI):560.1 $[M+1]^+$.

$^1$H NMR (400 MHz, $CDCl_3$)) $\delta$ 10.34 (s, 1H), 10.24 (s, 1H), 9.71 (s, 1H), 8.89 - 8.47 (m, 2H), 8.12 (s, 1H), 7.62 (d, *J*= 11.8 Hz, 1H), 3.75 (s, 2H), 3.35 (q, J =6.5 Hz, 2H), 2.84 (s, 2H), 2.62 (s, 3H), 2.47 - 2.35 (m, 8H), 1.03 (t, *J*= 6.5 Hz, 3H), 0.76-0.74 (m, 2H), 0.52-0.48 (m, 2H).

Examples 6, 10 and 11

In accordance with the method described in Examples 1 to 3, appropriate compounds were used as starting materials, accordingly, the following compounds were prepared:

$4^5$-((4-ethylpiperazin-1-yl)methyl)-$1^4$,$2^5$-difluoro-$1^2$,8-dimethyl-$1^1$H-5-oxa-3-aza-1 (6,1)-benzo[d]imidazola-2(4,2)-pyrimidina-4(2,4)-pyridinacyclooctaphane **6**;

$4^5$-((4-ethylpiperazin-1-yl)methyl)-$1^4$,$2^5$-difluoro-$1^2$,8-dimethyl-$1^1$H-3,6-diaza-1(6, 1)-benzo[d]imidazola-2(4,2)-pyrimidina-4(2,4)-pyridinacyclooctaphan-5-one **10**;
$4^5$-(4-ethylpiperazin-1-yl)-$1^4$,$2^5$-difluoro-$1^2$,8-dimethyl-$1^1$H-3,6-diaza-1(6,1)-benzo [d]imidazola-2(4,2)-pyrimidina-4(2,4)-pyridinacyclooctaphan-5-one **11.**

Test Example 1

**Determination of inhibitory effect on CDK4/Cyclin D1**

The determination of $IC_{50}$ of CDK4/Cyclin D1 was conducted according to the following process. A 96-well filter plate (Millipore MADVN6550) was used. The total volume was 0.1 ml, containing buffer solution A (20mM TRIS (tris[hydroxymethyl]aminomethane) (pH7.4), 50 mM NaCl, 1 mM dithiothreitol, 10 mM MgC12), 25 $\mu$M ATP (containing 0.25 $\mu$Ci[32P]ATP), 20 ng CDK4, 1 $\mu$g retinoblastoma protein and the test compound diluted in buffer solution A in an appropriate ratio. Buffer solution A alone without the test compound was used as the control without inhibitory effect. Buffer solution A containing excess EDTA was used to determine the background 32P level in the absence of enzymatic activity. All components except ATP were added to the wells, and the plate was shaked on a plate mixer for 2 minutes. [32P]ATP was added to initiate the reaction, and the plate was incubated at 25°C for 15 minutes. 0.1 ml of 20% trichloroacetic acid (TCA) was added to stop the reaction. The plate was kept at 4°C for at least 1 hour to allow the substrate to precipitate. The wells were washed five times with 0.2 ml of 10% TCA, and the binding of 32P was measured using a $\beta$ plate counter (wallac Inc., Gaithersburg, MD). $IC_{50}$ of the test compound was determined by median effect method (Chou, T-C and Talalay P. Applications of the median effect principle for the assessment of low-dose risk of carcinogens and for the quantitation of synergism and antagonism of chemotherapeutic agents. In New Avenues in Developmental Cancer Chemotherapy (Eds. Harrap, K.T. and Connors, T.A.), pp.37-64. Academic Press, New York, 1987).

Experimental method: Inhibitory activity on CDK4/6 kinase was determined by Caliper Mobility Shift method

1. Preparation of 1-fold kinase buffer solution:

1) Preparation of 1-fold CDK4 kinase buffer solution
800 $\mu$L of 1000 mM HEPES stock solution (pH 7.5) and 40 $\mu$L of 10% Triton X-100 stock solution were added to 39160 $\mu$L of ultra pure water, and the resulting solution was mixed well.
2) Preparation of 1-fold CDK6 kinase buffer solution
50 mL of 1000 mM HEPES stock solution (pH 7.5) and 50 $\mu$L of 30% Brij-35 stock solution were added to 949.95 mL of ultra pure water, and the resulting solution was mixed well.

2. Preparation of stop solution
25 mL of 4% Coating Reagent #3 (provided with the 12-sipper chip used in Caliper device) stock solution, 50 mL of 1000 mM HEPES stock solution (pH 7.5), 50 mL of 0.5 M EDTA stock solution and 0.25 mL of 30% Brij-35 stock solution were added to 374.75 mL of ultra pure water, and the resulting solution was mixed well.

3. Preparation of 2.5-fold kinase solution

1) Preparation of 2.5-fold CDK4/D3 kinase solution
7 $\mu$L of CDK4/D3 enzyme solution and 9 $\mu$L of 1M DTT stock solution were added to 1784 $\mu$L of 1-fold CDK4 kinase buffer solution, and the resulting solution was mixed well.
2) Preparation of 2.5-fold CDK6/D3 kinase solution
18 $\mu$L of CDK6/D3 enzyme solution and 14 $\mu$L of 1M DTT stock solution were added to 2768 $\mu$L of 1-fold CDK6 kinase buffer solution, and the resulting solution was mixed well.

4. Preparation of 2.5-fold polypeptide solution

1) Preparation of 2.5-fold CDK4/D3 polypeptide solution
10 $\mu$L of 100 mM ATP stock solution, 45 $\mu$L of 1M $MgCl_2$ stock solution and 45 $\mu$L of FAM-labelled polypeptide 8 were added to 1700 $\mu$L of 1-fold CDK4 kinase buffer solution, and the resulting solution was mixed well.
2) Preparation of 2.5-fold CDK6/D3 polypeptide solution
23 $\mu$L of 100 mM ATP stock solution, 75 $\mu$L of 1M $MgCl_2$ stock solution and 75 $\mu$L of FAM-labelled polypeptide 8 were added to 2827 $\mu$L of 1-fold CDK6 kinase buffer solution, and the resulting solution was mixed well.

5. Preparation of 5-fold test compound solution:

A 10 mM solution of the test compound in DMSO was diluted with DMSO to obtain a solution with a concentration of 50 μM, which was used as the stock solution. Said stock solution was subjected to 4-fold gradient dilution with DMSO to obtain solutions with a concentration of 12.5 μM, 3.125 μM, 0.78 μM, 0.195 μM, 0.0488 μM, 12.2 nM, 3 nM, 0.76 nM and 0.19 nM, respectively. Each of the resulting solution was 10-fold diluted with 1-fold kinase buffer solution to obtain a 5-fold compound solution.

6. CDK4/6 enzymatic reaction:

1) 5 μL of the prepared 5-fold test compound solution and 10 μL of the prepared 2.5-fold kinase solution were added to the corresponding wells in a 384-well plate, and the plate was incubated at room temperature for 10 minutes.
2) 10 μL of the prepared 2.5-fold polypeptide solution was added to the corresponding wells to start the enzymatic reaction, and the plate was incubated at 28°C for 5 hours.

7. Enzymatic assay:
25 μL of stop solution was added to each corresponding well to stop the reaction.
8. The data was read by the Caliper device, the inhibition rate was calculated by the following formula, and curve fitting was carried out by GraphPad5.0 software to obtain the $IC_{50}$ value.

| Example No. | CDK4 $IC_{50}$ (nM) | CDK6 $IC_{50}$ (nM) |
|---|---|---|
| LY2835219 | 1.2 | 2.2 |
| 1 | 0.70 | 0.48 |
| 2 | 0.49 | 0.35 |
| 3 | 15 | 27 |
| 4 | 0.78 | 1.13 |
| 5 | 21 | 14 |
| 7 | 1.5 | 2.5 |
| 8 | 8 | 10 |
| 9 | 5 | 12 |

Test Example 2

*In vitro* cell inhibitory activity of the compound of the present invention

Experimental method: Cell proliferation assay was carried out by BrdU method (BrdU cell proliferation assay kit, Cell Signaling Technology Co.)

1. Preparation of reagents and compounds

Preparation of 1-fold washing solution:

A stock washing solution (concentration: 20-fold) was diluted with ultra pure water to obtain the 1-fold washing solution.

Preparation of 1-fold detection antibody solution:

A BrdU detection antibody stock solution (concentration: 100-fold) was diluted with a detection antibody diluent to obtain the 1-fold detection antibody solution.

Preparation of 1-fold HRP-labelled secondary antibody solution:

A stock solution of HRP-labelled anti-mouse IgG antibody (concentration: 100-fold) was diluted with HRP-labelled

antibody diluent to obtain the 1-fold HRP-labelled secondary antibody solution.
10-fold BrdU solution:

A BrdU stock solution (concentration: 1000-fold) was diluted with the corresponding cell culture medium to obtain the 10-fold BrdU solution.

Preparation of test compound:

Preparation of test compound stock solution: 10 mM stock solution was prepared by 100% DMSO.

Preparation of gradient dilution solution of test compound: the 10 mM stock solution of test compound was subjected to 4-fold serial gradient dilution with DMSO to obtain solutions with a concentration of 2.5 mM, 625 μM, 156 μM, 39 μM, 9.8 μM and 2.5 μM, respectively. 2 μL of DMSO-diluted compound was added to 198 μL of culture medium containing 10% FBS to obtain 10-fold test compound, wherein the maximum concentration of the test compound was 100 μM, the DMSO concentration was 1%, with a total of 7 concentration gradients.
Preparation of culture medium:

MCF-7 culture medium: DMEM+10% FBS+0.01 mg/mL insulin

2. Experimental procedures

(1) Cells grown to 80% (in exponential growth phase) were digested with pancreatin, and were collected by centrifugation. MDA-MB-435S cells and U87MG cells were re-suspended in a FBS-free culture medium, counted and inoculated to a 96-well plate; MDA-MB-435S cells were inoculated at 3000 cells/well/81 μL, and U87MG cells were inoculated at 4000 cells/well/81 μL. MCF-7 cells were resuspended in a culture medium containing 1% FBS, counted and inoculated to a 96-well plate at 4000 cell/well/82 μL. The cells were incubated in a cell incubator at 37°C.
(2) After 24 hours of incubation, FBS (9 μL) was added to each well of the MDA-MB-435S cells and U87MG cells, and 8 μL of FBS was added to each well of the MCF-7 cells, so as to obtain a final FBS concentration of 10%.
(3) A different concentration of 10-fold test compound (10 μL) was added to each well to obtain a final concentration of test compound of 10 μM, 2.5 μM, 625 nM, 156 nM, 39 nM, 9.8 nM or 2.5 nM, respectively, with 3 repeated wells/group, and the cells were incubated at 37°C for 72 hours.
Solvent control: 0.1% DMSO
Blank control: only culture medium without cells
Normal cell control: normal cells without any treatment
(4) 10-fold BrdU solution (10 μL) was added to each well, and the cells were incubated in the incubator for 4 hours, followed by discarding the culture medium.
(5) Fixing/denaturation solution (10 μL) was added to each well, and the cells were incubated at room temperature for 30 minutes, followed by discarding the solution.
(6) 1-fold detection antibody solution (100 μL) was added to each well, the cells were incubated at room temperature for 1 hour, followed by discarding the solution, and the cells were washed with 1-fold washing solution (200 μL/well) three times.
(7) 1-fold HRP-labelled secondary antibody solution (100 μL) was added to each well, the cells were incubated at room temperature for 30 minutes, followed by discarding the solution, and the cells were washed with 1-fold washing solution (200 μL/well) three times.
(8) TMB substrate solution (100 μL) was added to each well, and the cells were incubated at room temperature for 30 minutes.
(9) Stop solution (100 μL) was added to each well, and the OD value at 450 nm was measured by a microplate reader.

3. Data processing

1)

$$\text{Cell survival rate (\%)} = (OD_{\text{test compound}} - OD_{\text{blank control}})/(OD_{\text{normal cell control}} - OD_{\text{blank control}}) \times 100\%,$$

$OD_{blank}$ control: blank control value; $OD_{normal\ cell\ control}$: normal cell control value;
2) The data was processed by GraphPad Prism 5 software to obtain the curve and $IC_{50}$ values.

| Example No. | MCF-7 $IC_{50}$ (nM) |
|---|---|
| LY2834219 | 120 |
| 1 | 142 |
| 2 | 42 |
| 3 | 4125 |
| 4 | 132 |

## Claims

**1.** A compound of formula (I):

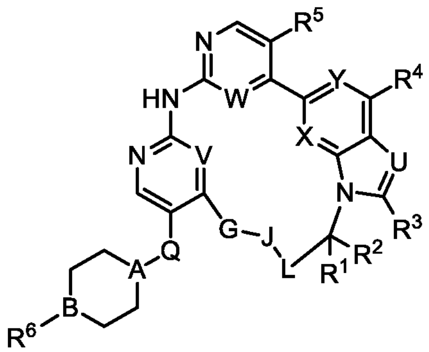

or a tautomer, mesomer, racemate, enantiomer, diastereomer thereof, or mixture thereof, or a pharmaceutically acceptable salt thereof,
wherein:

A and B are each independently selected from the group consisting of CH and N atom;
U, V, W, X and Y are each independently selected from the group consisting of CH and N atom;
Q is selected from the group consisting of a bond, $C_{1-4}$ alkylene and -C(=O)-, wherein the $C_{1-4}$ alkylene is optionally substituted by one or more substituents selected from the group consisting of $C_{1-4}$ alkyl, halo$C_{1-4}$ alkyl, $C_{1-4}$ alkoxy, halo$C_{1-4}$ alkoxy, halogen, amino, nitro, hydroxy and cyano;
G is selected from the group consisting of a bond, -O-, -N($R^7$)-, -C(=O)-, $C_{1-4}$ alkylene, $C_{2-4}$ alkenyl, -S-, -SO-, -$SO_2$- and 3 to 6 membered heterocyclyl, wherein the $C_{1-4}$ alkylene, $C_{2-4}$ alkenyl and 3 to 6 membered heterocyclyl are each independently optionally substituted by one or more substituents selected from the group consisting of $C_{1-4}$ alkyl, halo$C_{1-4}$ alkyl, $C_{1-4}$ alkoxy, halo$C_{1-4}$ alkoxy, halogen, amino, nitro, hydroxy and cyano;
J is selected from the group consisting of a bond, -O-, -N($R^7$)-, -C(=O)-, $C_{1-4}$ alkylene, $C_{2-4}$ alkenyl, -S-, -SO-, -$SO_2$- and 3 to 6 membered heterocyclyl, wherein the $C_{1-4}$ alkylene, $C_{2-4}$ alkenyl and 3 to 6 membered heterocyclyl are each independently optionally substituted by one or more substituents selected from the group consisting of $C_{1-4}$ alkyl, halo$C_{1-4}$ alkyl, $C_{1-4}$ alkoxy, halo$C_{1-4}$ alkoxy, halogen, amino, nitro, hydroxy and cyano;
L is selected from the group consisting of a bond, -C(=O)-, $C_{1-4}$ alkylene, $C_{2-4}$ alkenyl, -S-, -SO-, -$SO_2$- and 3 to 6 membered heterocyclyl, wherein the $C_{1-4}$ alkylene, $C_{2-4}$ alkenyl and 3 to 6 membered heterocyclyl are each independently optionally substituted by one or more substituents selected from the group consisting of $C_{1-4}$ alkyl, halo$C_{1-4}$ alkyl, $C_{1-4}$ alkoxy, halo$C_{1-4}$ alkoxy, halogen, amino, nitro, hydroxy and cyano;
$R^1$ is selected from the group consisting of H atom, $C_{1-4}$ alkyl, cyano, $C_{2-4}$ alkenyl and $C_{2-4}$ alkynyl, wherein the $C_{1-4}$ alkyl, $C_{2-4}$ alkenyl and $C_{2-4}$ alkynyl are each independently optionally substituted by one or more substituents selected from the group consisting of halogen, amino, nitro, hydroxy and cyano;
$R^2$ is selected from the group consisting of H atom and $C_{1-4}$ alkyl, wherein the $C_{1-4}$ alkyl is optionally substituted by one or more substituents selected from the group consisting of halogen, amino, nitro, hydroxy and cyano;
or, $R^1$ and $R^2$ together with the atom to which they are attached form a $C_{3-6}$ cycloalkyl or 3 to 6 membered

heterocyclyl, wherein the $C_{3-6}$ cycloalkyl or 3 to 6 membered heterocyclyl are each independently optionally substituted by one or more substituents selected from the group consisting of $C_{1-4}$ alkyl, halo$C_{1-4}$ alkyl, $C_{1-4}$ alkoxy, halo$C_{1-4}$ alkoxy, halogen, amino, nitro, hydroxy and cyano;
$R^3$ and $R^6$ are each independently selected from the group consisting of H atom and $C_{1-4}$ alkyl, wherein the alkyl is optionally substituted by one or more substituents selected from the group consisting of $C_{1-4}$ alkoxy, halo$C_{1-4}$ alkoxy, halogen, amino, nitro, hydroxy and cyano;
$R^4$ and $R^5$ are each independently selected from the group consisting of H atom, $C_{1-4}$ alkyl, halogen, hydroxy and $C_{1-4}$ alkoxy, wherein the $C_{1-4}$ alkyl and $C_{1-4}$ alkoxy are each independently optionally substituted by one or more substituents selected from the group consisting of halogen, amino, nitro, hydroxy and cyano; and
$R^7$ is selected from the group consisting of H atom and $C_{1-4}$ alkyl, wherein the alkyl is optionally substituted by one or more substituents selected from the group consisting of $C_{1-4}$ alkoxy, halo$C_{1-4}$ alkoxy, halogen, amino, nitro, hydroxy and cyano.

**2.** The compound of formula (I) according to claim 1, wherein -G-J-L- is selected from the group consisting of -NH-$CH_2$-$CH_2$-, -O-$CH_2$-$CH_2$-, -NH-C(=O)-$CH_2$- and -C(=O)-NH-$CH_2$-.

**3.** The compound of formula (I) according to claim 1 or 2, wherein Q is a bond or methylene.

**4.** The compound of formula (I) according to any one of claims 1 to 3, wherein V, X and Y are each independently CH.

**5.** The compound of formula (I) according to any one of claims 1 to 4, wherein W is an N atom.

**6.** The compound of formula (I) according to any one of claims 1 to 5, wherein $R^1$ and $R^2$ are each independently selected from the group consisting of H atom and $C_{1-4}$ alkyl, or $R^1$ and $R^2$ together with the atom to which they are attached form a $C_{3-6}$ cycloalkyl.

**7.** The compound of formula (I) according to any one of claims 1 to 6, wherein $R^3$ and $R^6$ are each independently a $C_{1-4}$ alkyl.

**8.** The compound of formula (I) according to any one of claims 1 to 7, wherein $R^4$ and $R^5$ are each independently a halogen, and preferably an F atom.

**9.** The compound of formula (I) according to any one of claims 1 to 8, selected from the group consisting of:

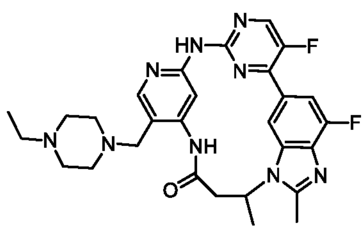,
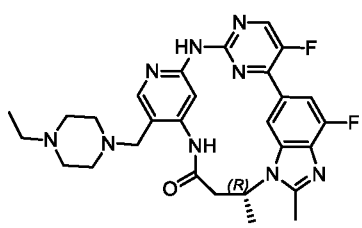,
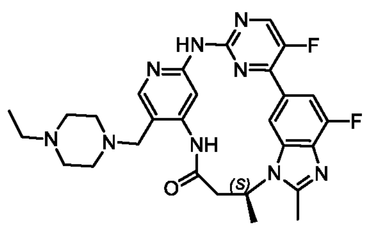,

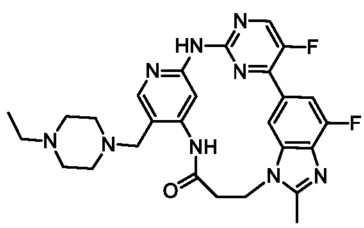,
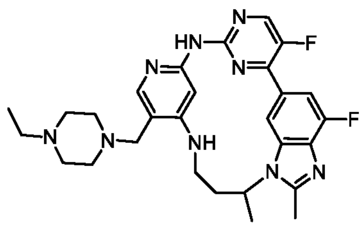,
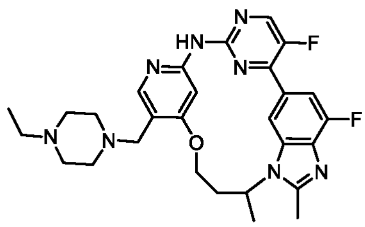,

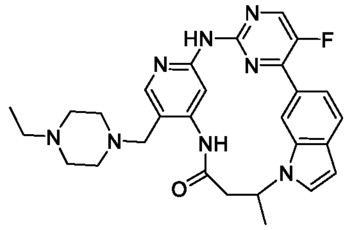,
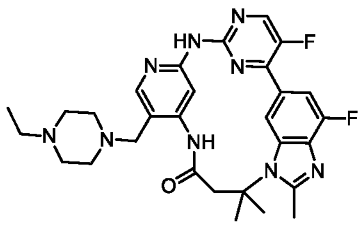,
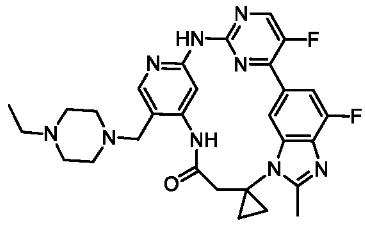,

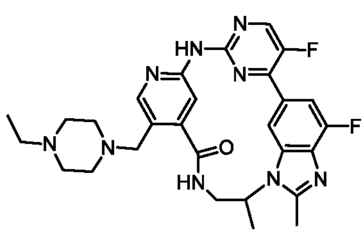
and
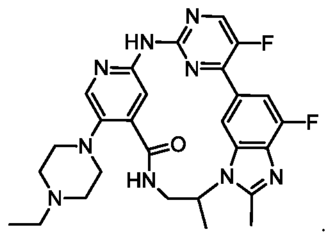.

**10.** A method for preparing the compound of formula (I) according to any one of claims 1 to 9, comprising a step of:

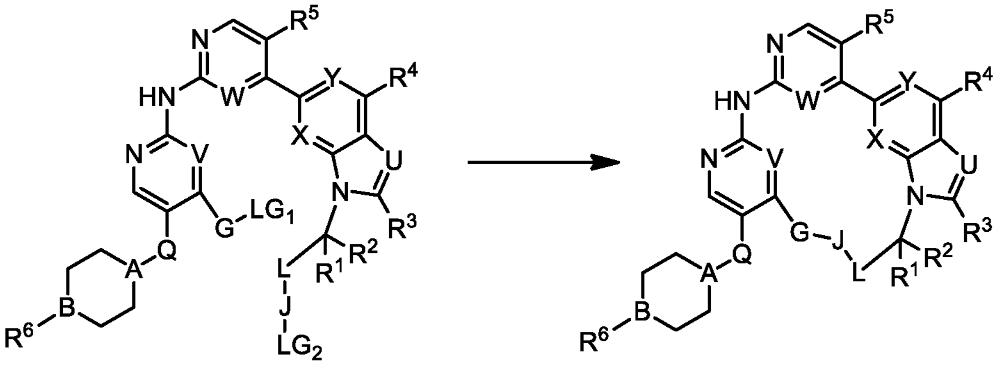

(IA) (I)

subjecting a compound of formula (IA) to an intramolecular cyclization reaction at the presence of a condensing agent to obtain the compound of formula (I),
wherein:

$LG_1$ and $LG_2$ are each independently a leaving group; and
A, B, U, V, W, X, Y, G, J, L, and $R^1$ to $R^6$ are as defined in claim 1.

**11.** The method according to claim 10, wherein the condensing agent is selected from the group consisting of 2-(7-azabenzotriazol-1-yl)-*N*,*N*,*N'*,*N'*-tetramethyluronium hexafluorophosphate, 1-(3-dimethylaminopropyl)-3-ethylcarbodiimide hydrochloride, *N,N'*-dicyclohexylcarbodiimide, *N*,*N'*-diisopropylcarbodiimide, *O*-(benzotriazol-1-yl)-*N*,*N*,*N*,*N'*-tetramethyluronium tetrafluoroborate, 1-hydroxybenzotriazole, 1-hydroxy-7-azobenzotriazole, *O*-(benzotriazol-1-yl)-*N*,*N*,*N'*,*N'*-tetramethyluronium hexafluorophosphate, 2-(7-azobenzotriazole)-*N*,*N*,*N',N'*-tetramethyluronium hexafluorophosphate, benzotriazol-1-yloxytris(dimethylamino)phosphonium hexafluorophosphate and benzotriazol-1-yl-oxytripyrrolidinophosphonium hexafluorophosphate, and preferably 2-(7-azabenzotriazol-1-yl)-*N*,*N*,*N',N'*-tetramethyluronium hexafluorophosphate; and
$LG_1$ and $LG_2$ are each independently selected from the group consisting of H atom, OH, halogen, mesylate, triflate and tosylate; preferably, $LG_1$ and $LG_2$ are each independently selected from the group consisting of H atom, OH, Cl atom and Br atom; and more preferably, $LG_1$ is a H atom and $LG_2$ is OH.

**12.** The method according to claim 10 or 11, wherein G is -NH-, $LG_1$ is a H atom, J is -C(=O)-, and $-LG_2$ is OH.

**13.** A pharmaceutical composition, comprising a therapeutically effective amount of the compound of formula (I) according to any one of claims 1 to 9, and pharmaceutically acceptable carrier(s), diluent(s) or excipient(s), and

preferably further comprising one or more additional anti-tumor agents, anti-inflammatory agents, immunosuppressants and/or immune checkpoint inhibitors.

**14.** Use of the compound of formula (I) according to any one of claims 1 to 9 or the pharmaceutical composition according to claim 13 in the preparation of a medicament for treating a CDK-related disease, wherein the CDK-related disease preferably is selected from the group consisting of cancer, inflammation, viral infection, cardiac hypertrophy and HIV

**15.** The use according to claim 14, wherein the cancer is selected from the group consisting of bladder cancer, head and neck cancer, breast cancer, stomach cancer, ovarian cancer, colon cancer, lung cancer, brain cancer, laryngeal cancer, lymphatic system cancer, hematopoietic system cancer, genitourinary tract cancer, gastrointestinal cancer, ovarian cancer, prostate cancer, gastric cancer, bone cancer, small cell lung cancer, glioma, colorectal cancer and pancreatic cancer;
the inflammation is related to rheumatoid arthritis, lupus, type 1 diabetes, diabetic nephropathy, multiple sclerosis, glomerulonephritis, chronic inflammation and organ transplant rejection;
the viral infection is related to HIV virus, human papilloma virus, herpes virus, pox virus, Epstein-Barr virus, Sindbis virus or adenovirus.

**INTERNATIONAL SEARCH REPORT**

International application No.
**PCT/CN2019/124575**

**A. CLASSIFICATION OF SUBJECT MATTER**

C07D 498/04(2006.01)i; A61P 35/00(2006.01)i; A61K 31/519(2006.01)i

According to International Patent Classification (IPC) or to both national classification and IPC

**B. FIELDS SEARCHED**

Minimum documentation searched (classification system followed by classification symbols)

C07D; A61P; A61K

Documentation searched other than minimum documentation to the extent that such documents are included in the fields searched

Electronic data base consulted during the international search (name of data base and, where practicable, search terms used)

CNPAT; CNKI; WPI; EPODOC; CA; STN REGISTRY; STN CAPLUS; 凯复制药; 蛋白依赖性激酶; CDK; 癌症; 肿瘤; 艾滋病; AIDS; HIV; Camphor pharmaceuticals; cyclin-dependent kinase; Cancer; Tumor.

**C. DOCUMENTS CONSIDERED TO BE RELEVANT**

| Category* | Citation of document, with indication, where appropriate, of the relevant passages | Relevant to claim No. |
|---|---|---|
| A | CN 101360751 A (S. BIO PTE LTD.) 04 February 2009 (2009-02-04) claims 1-138, description, tables 1-6 | 1-15 |
| A | CN 102946732 A (THE REGENTS OF THE UNIVERSITY OF COLORADO) 27 February 2013 (2013-02-27) claims 1-19 | 1-15 |
| A | CN 107708719 A (ONKURE INC.) 16 February 2018 (2018-02-16) claims 1-16 | 1-15 |
| A | WO 2018177899 A1 (BAYER AKTIENGESELLSCHAFT) 04 October 2018 (2018-10-04) abstract, claims 1-23, description, compounds in tables 1-4 | 1-15 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **26 February 2020** | **10 March 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2019/124575**

| Patent document cited in search report | Publication date (day/month/year) | Patent family member(s) | Publication date (day/month/year) |
|---|---|---|---|
| CN 101360751 A | 04 February 2009 | KR 20140117679 A | 07 October 2014 |
| | | DE 602006014579 D1 | 08 July 2010 |
| | | US 2012142680 A1 | 07 June 2012 |
| | | AT 469158 T | 15 June 2010 |
| | | HK 1123282 A1 | 06 March 2015 |
| | | ES 2506040 T3 | 13 October 2014 |
| | | CA 2629443 C | 13 December 2016 |
| | | KR 20080086443 A | 25 September 2008 |
| | | TW 200736262 A | 01 October 2007 |
| | | DK 1951729 T3 | 06 October 2014 |
| | | KR 20140037256 A | 26 March 2014 |
| | | US 8415338 B2 | 09 April 2013 |
| | | AU 2006316071 B2 | 17 February 2011 |
| | | US 2009258886 A1 | 15 October 2009 |
| | | KR 101499594 B1 | 09 March 2015 |
| | | TW 201418263 A | 16 May 2014 |
| | | US 2012196855 A1 | 02 August 2012 |
| | | KR 20080090390 A | 08 October 2008 |
| | | MY 148072 A | 28 February 2013 |
| | | EP 1951729 A1 | 06 August 2008 |
| | | AU 2006316072 B2 | 22 December 2011 |
| | | CA 2629455 C | 03 January 2017 |
| | | US 8143255 B2 | 27 March 2012 |
| | | JP 5380073 B2 | 08 January 2014 |
| | | JP 2009517344 A | 30 April 2009 |
| | | CY 1116156 T1 | 08 February 2017 |
| | | DK 1951730 T3 | 27 September 2010 |
| | | US 8153632 B2 | 10 April 2012 |
| | | PT 1951730 E | 27 August 2010 |
| | | WO 2007058627 A1 | 24 May 2007 |
| | | CA 2629443 A1 | 24 May 2007 |
| | | EP 1951729 A4 | 23 September 2009 |
| | | AU 2006316071 A1 | 24 May 2007 |
| | | WO 2007058628 A1 | 24 May 2007 |
| | | TW 200738735 A | 16 October 2007 |
| | | PT 1951729 E | 02 October 2014 |
| | | TW I525096 B | 11 March 2016 |
| | | TW I407961 B | 11 September 2013 |
| | | AU 2006316072 A1 | 24 May 2007 |
| | | KR 101576159 B1 | 09 December 2015 |
| | | KR 20140037257 A | 26 March 2014 |
| | | KR 101568801 B1 | 12 November 2015 |
| | | US 2009075999 A1 | 19 March 2009 |
| | | TW I506029 B | 01 November 2015 |
| | | CA 2629455 A1 | 24 May 2007 |
| | | CN 101365703 B | 06 November 2013 |
| | | JP 2009515954 A | 16 April 2009 |
| | | BR PI0618552 A2 | 06 September 2011 |
| | | ES 2346791 T3 | 20 October 2010 |
| CN 102946732 A | 27 February 2013 | EP 2575467 B1 | 06 July 2016 |

Form PCT/ISA/210 (patent family annex) (January 2015)

**INTERNATIONAL SEARCH REPORT**
**Information on patent family members**

International application No.
**PCT/CN2019/124575**

| Patent document cited in search report | | | Publication date (day/month/year) | Patent family member(s) | | | Publication date (day/month/year) |
|---|---|---|---|---|---|---|---|
| | | | | KR | 102028850 | B1 | 04 October 2019 |
| | | | | HK | 1181613 | A1 | 24 July 2015 |
| | | | | ES | 2594500 | T3 | 20 December 2016 |
| | | | | PT | 2575467 | T | 14 October 2016 |
| | | | | JP | 2013528182 | A | 08 July 2013 |
| | | | | US | 8754050 | B2 | 17 June 2014 |
| | | | | CA | 2800958 | C | 04 September 2018 |
| | | | | BR | 112012030193 | A2 | 29 September 2015 |
| | | | | US | 9422340 | B2 | 23 August 2016 |
| | | | | HU | E030812 | T2 | 29 May 2017 |
| | | | | AU | 2011258110 | A1 | 10 January 2013 |
| | | | | WO | 2011150283 | A1 | 01 December 2011 |
| | | | | JP | 5931850 | B2 | 08 June 2016 |
| | | | | LT | 2575467 | T | 10 January 2017 |
| | | | | EP | 2575467 | A4 | 15 October 2014 |
| | | | | CA | 2800958 | A1 | 01 December 2011 |
| | | | | US | 2013203681 | A1 | 08 August 2013 |
| | | | | DK | 2575467 | T3 | 26 September 2016 |
| | | | | KR | 20180081153 | A | 13 July 2018 |
| | | | | MX | 2012013507 | A | 12 March 2013 |
| | | | | KR | 20130123301 | A | 12 November 2013 |
| | | | | AU | 2011258110 | B2 | 16 July 2015 |
| | | | | US | 2015010541 | A1 | 08 January 2015 |
| | | | | PL | 2575467 | T3 | 31 October 2017 |
| | | | | CN | 102946732 | B | 26 November 2014 |
| | | | | EP | 2575467 | A1 | 10 April 2013 |
| | | | | RU | 2012157293 | A | 10 July 2014 |
| | | | | RU | 2565076 | C2 | 20 October 2015 |
| CN | 107708719 | A | 16 February 2018 | JP | 2019523750 | A | 29 August 2019 |
| | | | | WO | 2017201278 | A1 | 23 November 2017 |
| | | | | TW | 201742871 | A | 16 December 2017 |
| | | | | IL | 263159 | D0 | 31 December 2018 |
| | | | | CA | 3025045 | A1 | 23 November 2017 |
| WO | 2018177899 | A1 | 04 October 2018 | CA | 3057892 | A1 | 04 October 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015101293 A1
- WO 2016015605 A1
- WO 2016194831 A1
- WO 2008079933 A2


### Non-patent literature cited in the description


- **MALLINSON, J. ; COLLINS, I.** Macrocycles in new drug discovery. *Future Med. Chem.,* 2012, vol. 4, 1409-1438
- **KWITKOWSKI VE ; PROWELL TM ; IBRAHIM A et al.** FDA approval summary: temsirolimus as treatment for advanced renal cell carcinoma. *Oncologist,* 2010, vol. 15 (4), 428-435
- **MCDONALD E ; WORKMAN P ; JONES K.** Inhibitors of the HSP90 molecular chaperone: attacking the master regulator in cancer. *Curr. Top. Med. Chem.,* 2006, vol. 6 (11), 1091-1107
- **HART S ; GOH KC ; NOVOTNY-DIERMAYR V et al.** SB 1518, a novel macrocyclic pyrimidinebased JAK2 inhibitor for the treatment of myeloid and lymphoid malignancies. *Leukemia,* 2011, vol. 25 (11), 1751-1759
- **WILLIAM AD ; LEE ACH ; GOH KC et al.** Discovery of kinase spectrum selective macrocycle (16E)-14-methyl-20oxa5,7,14,26-tetraazatetracyclo [19.3.1.1(2,6).1(8,12)]heptacosa1(25 ),2(26),3,5,8( 27),9,11,16,21,23-decaene (SB1317/TG02), a potent inhibitor of cyclin dependent kinases (CDKs), janus kinase 2 (JAK2), and Fms-like tyrosine kinase-3 (FLT3) for the treatment of cancer. *J. Med. Chem.,* 2012, vol. 55 (1), 169-196
- **STUPP R ; VAN DEN BENT MJ ; ERRIDGE SC et al.** Cilengitide in newly diagnosed glioblastoma with MGMT promoter methylation: Protocol of a multicenter, randomized, open-label, controlled phase III trial (CENTRIC). *J. Clin. Oncol.,* 2010, vol. 28 (15s), TPS152
- **HIRAI H ; TAKAHASHI-SUZIKI I ; SHIMOMURA T et al.** Potent anti-tumor activity of a macrocycle-quinoxalinone class pan-Cdk inhibitor in vitro and in vivo. *Invest. New Drugs,* 2011, vol. 29 (4), 534-543
- Applications of the median effect principle for the assessment of low-dose risk of carcinogens and for the quantitation of synergism and antagonism of chemotherapeutic agents. **CHOU, T-C ; TALALAY P.** In New Avenues in Developmental Cancer Chemotherapy. Academic Press, 1987, 37-64